(19) Europäisches Patentamt
European Patent Office
Office européen des brevets

(11) Publication number: 0 429 304 A2

# (12) EUROPEAN PATENT APPLICATION

(21) Application number: 90312692.8

(51) Int. Cl.5: C07D 215/56, A61K 31/47

(22) Date of filing: 21.11.90

(30) Priority: 21.11.89 JP 302634/89

(43) Date of publication of application:
29.05.91 Bulletin 91/22

(84) Designated Contracting States:
AT BE CH DE DK ES FR GB GR IT LI LU NL SE

(71) Applicant: SHIONOGI SEIYAKU KABUSHIKI KAISHA trading under the name of SHIONOGI & CO. LTD.
1-8, Doshomachi 3-chome Chuo-ku
Osaka 541(JP)

(72) Inventor: Imose, Jun
4289-7 Ishibe, Ishibe-cho
Koga-gun, Shiga(JP)
Inventor: Kataoka, Takahiro
90 Kitanakakoji, Ritto-cho
Kurita-gun, Shiga(JP)
Inventor: Hidaka, Shigetada
1129-6 Mushiyono, Minakuchi-cho
Koga-gun, Shiga(JP)
Inventor: Ueda, Kazuo
1249 Shinjo-cho, Oaza, Seki-cho
Suzuka-gun, Mie(JP)
Inventor: Ogata, Masaru
8-20-8 Sumiyoshiyamate, Higashinada-ku
Kobe, Hyogo(JP)
Inventor: Takahashi, Toshio
9-21-306 Kumano-cho
Nishinomiya, Hyogo(JP)

(74) Representative: Hardisty, David Robert et al
BOULT, WADE & TENNANT 27 Furnival Street
London EC4A IPQ(GB)

## (54) Pyridone-carboxylic acid derivatives and their use as veterinary medicines.

(57) A veterinary composition for the treatment or prophylaxis of infectious diseases caused by infectious microorganisms which comprises at least one pyridone-carboxylic acid compound of the formula:

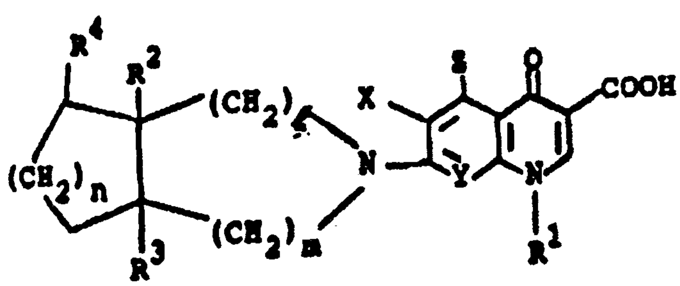

wherein
$R^1$ is lower alkyl, halo(lower)alkyl, cyclo(lower) alkyl or substituted or unsustituted phenyl;
$R^2$ and $R^3$ are each hydrogen, hydroxyl, lower alkoxy, halogen or a group of the formula: $-CH_2N(R^5)R^6$ (in which $R^5$ and $R^6$ are each hydrogen, lower alkyl, lower alkenyl or hydroxy(lower)alkyl);
$R^4$ is hydrogen, oxo, hydroxyl, lower alkoxy, halogen or a group of the formula: $-CH_2N(R^7)R^8$ (in which $R^7$ and $R^8$ are each hydrogen or lower alkyl;
X is hydrogen or halogen;
Y is CQ (in which Q is hydrogen or halogen) or nitrogen or, when taken together with $R^1$, may form a group of

EP 0 429 304 A2

the formula: $=C-OCH_2C(R^9)H-$ (in which $R^9$ is hydrogen or lower alkyl and the carbon atom to which $R^9$ is attached links to the nitrogen atom in the pyridone ring); the compound themselves and a process for their production.

# PYRIDONE-CARBOXYLIC ACID DERIVATIVES AND THEIR USE AS VETERINARY MEDICINES

The present invention relates to novel pyridone-carboxylic acid derivatives and their use as veterinary medicines for treatment and prevention of infectious diseases, particularly respiratory infections caused by infectious microorganisms such as mycoplasma.

A number of pyridone-carboxylic acid derivatives are available on the market as pharmaceutical drugs for human beings, and many studies and researches to improve their antimicrobial potency, antimicrobial spectra and intracorporeal behaviors (e.g. distribution, metabolism, excretion) are still carried out extensively. On the other hand, studies and researches on the use of pyridone-carboxylic acid derivatives a veterinary medicines are relatively few.

Among pyridone-carboxylic acid derivatives, some quinolone compounds are reported to be useful for treatment and prevention of infections with microorganisms, particularly as anti-mycoplasma agents, in animals. Examples of such quinolone compounds are 6-fluoro-3-quinolonecarboxylic acids having a piperazinyl group at the 7-position, which are representable by the formula (JP-A-62-22716):

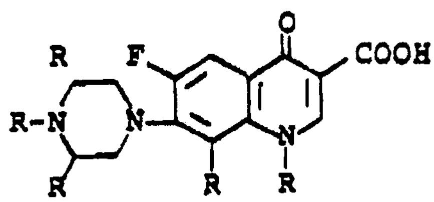

wherein R is a hydrogen atom, a lower alkyl group or the like, 6-fluoro-3-quinolonecarboxylic acids having a piperazinyl group at the 7-position and a six-membered ring condensed at the 1-, 8- and 9-positions, which are representable by the formula (JP-A-60-18014):

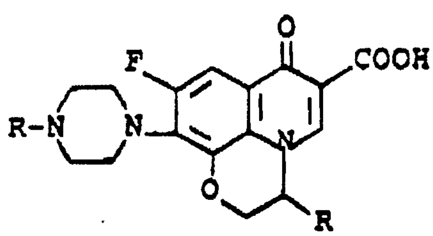

wherein R is as defined above, 6-halo-3-quinolonecarboxylic acids having a cyclopropyl group at the 1-position and a nitrogen-containing group at the 7-position, which are representable by the formula (WO086/0663):

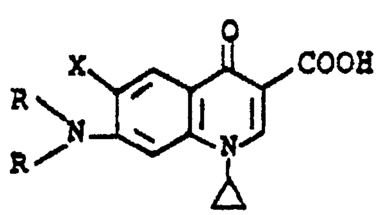

wherein R is as defined above, etc.

The pyridone-carboxylic acid derivatives according to the invention are much different from the above known quinolone compounds in chemical structure and show high anti-mycoplasma activity.

Mycoplasma is one of the microorganisms having no cell wall and known to be the principal causative micro organism in respiratory system infections, cephalomeningitis, epicardium, arthritis, etc. in human beings and animals. For treatment or prevention of such infectious diseases, tetracyclins and macrolide

antibiotics have so far been employed. In recent years, however, drug-resistant strains, especially to macrolide antibiotics are frequently produced, and the appearance of any anti-mycoplasma agent, particularly effective against drug-resistant strains, is highly demanded.

A main object of the present invention is to provide a veterinary medicine showing a strong antimicrobial activity against various pathogenic microorganisms, particularly against mycoplasma including their drug-resistant strains.

As the result of an extensive study, it has been found that certain pyridone-carboxylic acid compounds show a strong antimicrobial activity against various pathogenic microorganisms causing infectious diseases to animals such as domestic animals or fishes, particularly mycoplasma including their drug-resistant strains, especially their macrolide antibiotic-resistant strains. The present invention is based on the above finding.

According to the present invention, there is provided a veterinary medicinal composition comprising as an active ingredient a pyridone-carboxylic acid compound of the formula:

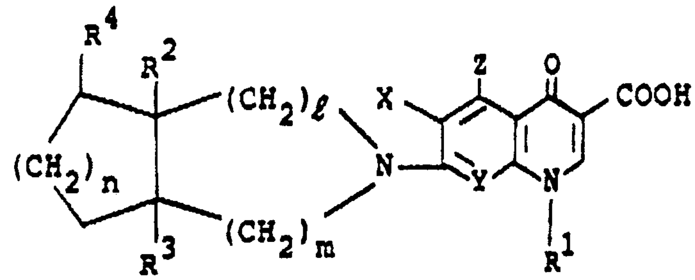

(I)

wherein

$R^1$ is lower alkyl, halo(lower)alkyl, cyclo(lower)alkyl or substituted or unsustituted phenyl;

$R^2$ and $R^3$ are each hydrogen, hydroxyl, lower alkoxy, halogen or a group of the formula: $-CH_2N(R^5)R^6$ (in which $R^5$ and $R^6$ are each hydrogen, lower alkyl, lower alkenyl or hydroxy(lower)alkyl);

$R^4$ is hydrogen, oxo, hydroxyl, lower alkoxy, halogen or a group of the formula: $-CH_2N(R^7)R^8$ (in which $R^7$ and $R^8$ are each hydrogen or lower alkyl);

X is hydrogen or halogen;

Y is CQ (in which Q is hydrogen or halogen) or nitrogen or, when taken together with $R^1$, may form a group of the formula: $=C-OCH_2C(R^9)H-$ (in which $R^9$ is hydrogen or lower alkyl and the carbon atom to which $R^9$ is attached links to the nitrogen atom in the pyridone ring);

Z is hydrogen or amino;

$\ell$ is an integer of 1 or 2;

m is an integer of 0 or 1; and

n is an integer of 1 or 2,

or a salt thereof.

In the above significances, the term "lower" is intended to mean a group having not more than 8 carbon atoms, particularly not more than 5 carbon atoms, unless otherwise indicated. When it is used in connection with a cyclic group, the cyclic group may have 3 to 8 carbon atoms, particularly 3 to 6 carbon atoms. Thus, the term "lower alkyl" includes methyl, ethyl, propyl, isopropyl, butyl, isobutyl, t-butyl, pentyl, hexyl, peptyl, octyl, etc., and methyl, ethyl, butyl, isobutyl, t-butyl, etc. are particularly preferred. As the term "cyclo(lower)alkyl", there may be exemplified cyclopropyl, cyclopentyl, cyclohexyl, cycloheptyl, cyclooctyl, etc. Of these, cyclopropyl, cyclopentyl and cyclohexyl are preferred. The term "halo(lower)alkyl" includes chloromethyl, trifluoromethyl, fluoroethyl, chloropropyl, fluorobutyl, etc. The term "lower alkoxy" includes methoxy, ethoxy, propoxy, butoxy, isobutoxy, etc. The term "halogen" includes fluorine, chlorine, bromine, etc. The "substituted or unsubstituted phenyl" may be, for instance, phenyl or phenyl substituted with lower alkyl, lower alkoxy or halogen, and its specific examples are phenyl, tolyl, xylyl, methoxyphenyl, fluorophenyl, difluorophenyl, dichlorophenyl, etc.

The pyridone-carboxylic acid compound (I) may be in a free or salt form. When it is in a salt form, there may be exemplified an acid addition salt (e.g. hydrochloride, hydrobromide, hydroiodide, sulfate, nitrate, phosphate, acetate, maleate, fumarate, citrate, tartrate), an alkali metal or alkaline earth metal salt (e.g. sodium salt, potassium salt, calcium salt, barium salt), ammonium salt, an organic amine salt (e.g. ethanolamine salt, N,N-dialkylethanolamine salt), etc.

The pyridone-carboxylic acid compound (I) can be produced according to the following reaction

scheme:

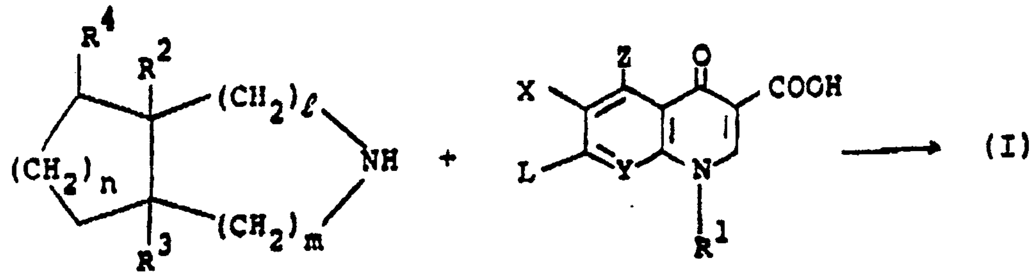

wherein $R^1$, $R^2$, $R^3$, $R^4$, X, Y, Z, ℓ, m and n are each as defined above and L is a reactive group such as halogen (e.g. chloride, bromine) or sulfonyloxy (e.g. methanesulfonyloxy, benzenesulfonyloxy, toluenesulfonyloxy).

The above reaction between the quinolonecarboxylic acid (II) and the azabicycloalkane (III) is usually performed in a liquid medium (e.g. water, methanol, ethanol, ether, acetonitrile, dimethylsulfoxide, dimethylformamide) at a temperature of room temperature (e.g. 15°C) to about 200°C, preferably from about 80 to 120°C or under reflux for about 1 to several hours. In order to accelerate the reaction, a base (e.g. triethylamine, pyridine, DBU (1,8-diazabicyclo[5.4.0]undec-7-ene) may be added to the reaction system.

When an amino group is present in the azabicycloalkane (III), the amino group may be protected by a per se conventional manner, for instance, formation of a salt. After the above reaction is accomplished, the protective group on the amino group may be eliminated by a per se conventional procedure, e.g. treatment with a base (e.g. sodium hydroxide, potassium hydroxide) or an acid (e.g. hydrochloric acid, acetic acid) in an inert solvent (e.g. water, ethanol, acetic acid) at room temperature to the reflux temperature.

The quinolonecarboxylic acid (II) as one of the starting compounds may be produced by conventional methods as dscribed in JP-A-61-2252 or JP-A-57-46986. The azabicycloalkane (III) may be also produced by conventional methods.

Some typical embodiments of preparation of the azabicycloalkane (III) as well as the objective pyridonecarboxylic acid compound (I) are illustratively shown in the following Reference Examples and Examples wherein the abbreviations show the following meanings: Tr: trityl; Me: methyl; Et: ethyl; n-Pr: n-propyl; Et-OH: hydroxyethyl; Ac: acetyl; Boc: t-butoxy; Ms: mesyl; Cbz: carbobenzoxy, etc. With respect to the $^1$H-NMR data, measurement was made using as the internal standard DSS in case of the solvent being $D_2O$ or TMS in case of the solvent being other than $D_2O$.

Reference Example 1

Preparation of (1R*,5S*)-1-methylaminomethyl-3-azabicyclo[3.3.0]octane hydrochloride (III-1):-

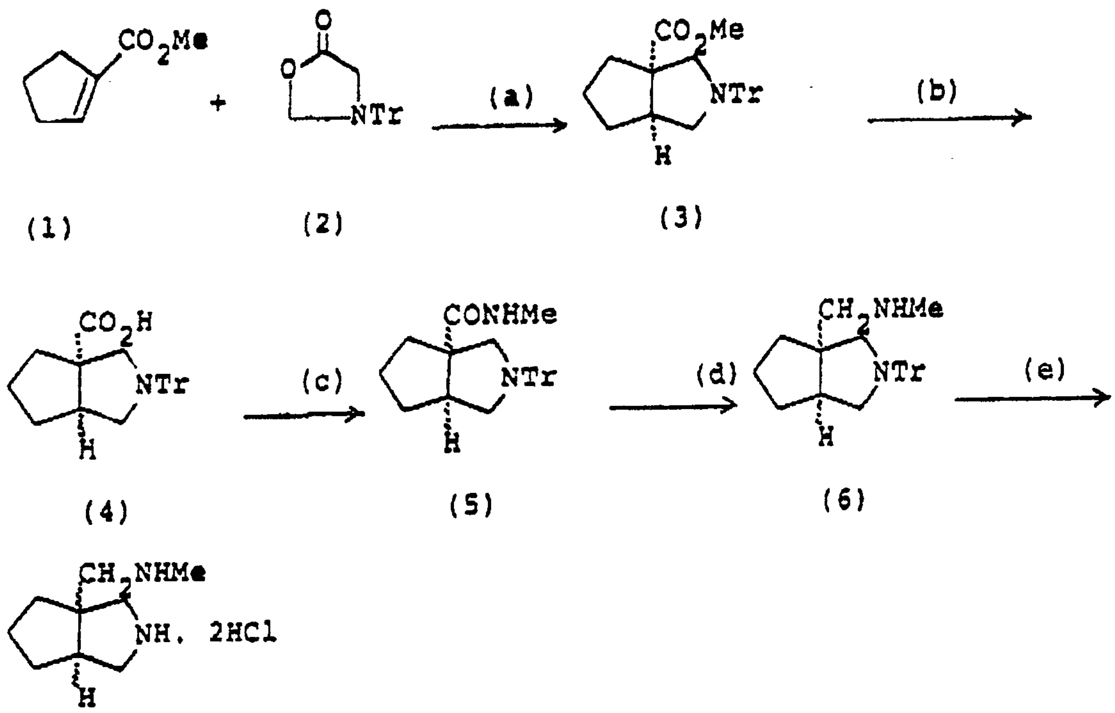


(1) (2) (3) (4) (5) (6) (III-1)

(a) A solution of methyl 2-cyclopentenecarboxylate (compound (1)) (J.Org.Chem., 35, p.3352 (1970)) (4.5 g) and 3-trityl-5-oxazolidinone (compound (2)) (11.8 g) in dry toluene (100 ml) is refluxed in an oil bath for 45 hours. Toluene is removed by distillation. The residue is chromatographed (eluent: methylene chloride/n-hexane = 1/2 (v/v)) on silica gel to give the compound (3) (3.9 g) as an oil.
IR (film): 1720, 1595 $cm^{-1}$
$^1$H-NMR ($CDCl_3$) $\delta$ (ppm): 1.40 - 3.00 (11H, m), 3.67 (3H, s), 7.00 - 7.60 (15H, m).

(b) The compound (3) (2.23 g) is added to a mixture of 20 % aqueous sodium hydroxide (11 ml) and methanol (46 ml), and the resultant mixture is refluxed for 2 hours, followed by removal of methanol. The residue is neutralized with acetic acid under ice-cooling and extracted with methylene chloride. The extract is washed with water, dried over sodium sulfate and concentrated. The residue is recrystallized from isopropyl ether to give the compound (4) (1.8 g). m.p., 221 - 222 °C (decomp.).
IR (nujol): 3100 - 2500, 1685 $cm^{-1}$.
$^1$H-NMR ($CDCl_3$) $\delta$ (ppm): 1.20 - 3.05 (11H, m), 7.00 - 7.55 (15H, m), 10.9 (1H, br).

(c) To a solution of the compound (4) (7.7 g) in dry tetrahydrofuran (77 ml), triethylamine (3.1 ml) is added, and the resultant mixture is ice-cooled. Ethyl chloroformate (2.0 ml) is dropwise added thereto, followed by stirring for 5 minutes. A 40 % methanolic solution of methylamine (10 ml) is added thereto at once, followed by stirring for 30 minutes. The reaction mixture is combined with ethyl acetate and a saturated sodium chloride solution, and the organic layer is seprated from the aqueous layer, washed with a saturated sodium chloride solution, dried over sodium sulfate and concentrated under reduced pressure. The residue is dissolved in ethyl acetate, dried over sodium sulfate and concentrated to give the compound (5) (8.3 g) as an oil.
$^1$H-NMR ($CDCl_3$) $\delta$ (ppm): 1.25 - 1.38 (3H, m), 1.52 - 1.70 (3H, m), 1.97 (1H, d, J = 9.8 Hz), 2.05 (1H, m), 2.80 - 2.95 (3H, m) 2.93 (3H, d, J = 4.9 Hz), 6.49 (1H, brd, J = 4.9 Hz), 7.14 - 7.45 (15H, m).

(d) The compound (5) (8.3 g) is dissolved in dry toluene (83 ml) under heating (40 °C) and a 70 % toluene solution of sodium hydrogen bis(2-methoxyethoxy)ammonium (17 ml) is dropwise added thereto, followed by stirring at 80 °C for 10 minutes. After cooling, the mixture is poured into ice-water, extracted with ethyl acetate, washed with a saturated aqueous potassium sodium tartarate solution and a saturated aqueous sodium chloride solution and dried over sodium sulfate. Removal of the solvent under reduced pressure gives the compound (6) (7.6 g) as an oil.
$^1$H-NMR ($CDCl_3$) $\delta$ (ppm): 1.45 - 1.80 (6H, m), 2.02 (3H, m), 2.17 (1H, d, J = 9.8 Hz), 2.25 (1H, d, J = 9.8 Hz), 2.37 (1H, m), 2.43 (3H, s), 2.53 (1H, d, J = 10.7 Hz), 2.60 (1H, d, J = 10.7 Hz), 7.12 (3H, t, J = 7.4 Hz), 7.24 (6H, t, J = 7.4 Hz), 7.48 (6H, d, J = 7.4 Hz).

(e) The compound (6) (7.6 ) is dissolved in methanol (38 mol) under heating (40° C) and allowed to cool to room temperature. A methanolic solution of 10 % hydrogen chloride (38 ml) is added to thereto, and the mixture is stirred for 1 hour. The solvent is removed under reduced pressure, and the residue is combined with ethyl acetate. The precipitated crystals are collected by filtration to give the compound (III-1) (2.5 g). $^1$H-NMR ($D_2O$) δ (ppm): 1.60 (1H, m), 1.76 - 1.81 (4H, m), 1.92 (1H, m), 2.64 (1H, m), 2.78 (3H, s), 3.09 (1H, dd, J = 12.2 Hz, J = 6.3 Hz), 3.21 (1H, d, J = 12.7 Hz), 3.29 (1H, d, J = 12.7 Hz), 3.50 (1H, d, J = 12.7 Hz), 3.59 (1H, dd, J = 12.7 Hz, J = 8.3 Hz).

In the same manner as above but using ethylamine in place of methylamine in the step (c), there is obtained the following compound as an oil:

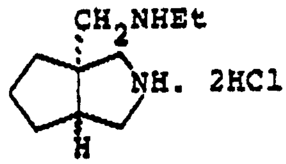

(III-1A)

$^1$H-NMR ($D_2O$, DSS) δ (ppm): 1.31 (3H, t, J = 7.0 Hz), 1.54 - 1.98 (6H, m), 2.62 (1H, m), 3.01 - 3.30 (6H, m), 3.51 (1H, d, J = 12.5 Hz), 3.57 (1H, dd, J = 12.5 Hz, J = 8.5 Hz).

Reference Example 2

Preparation of (1R*,2R*,6S*)-2-methylamino-7-azabicyclo[4.3.0]nonane hydrochloride (III-2):-

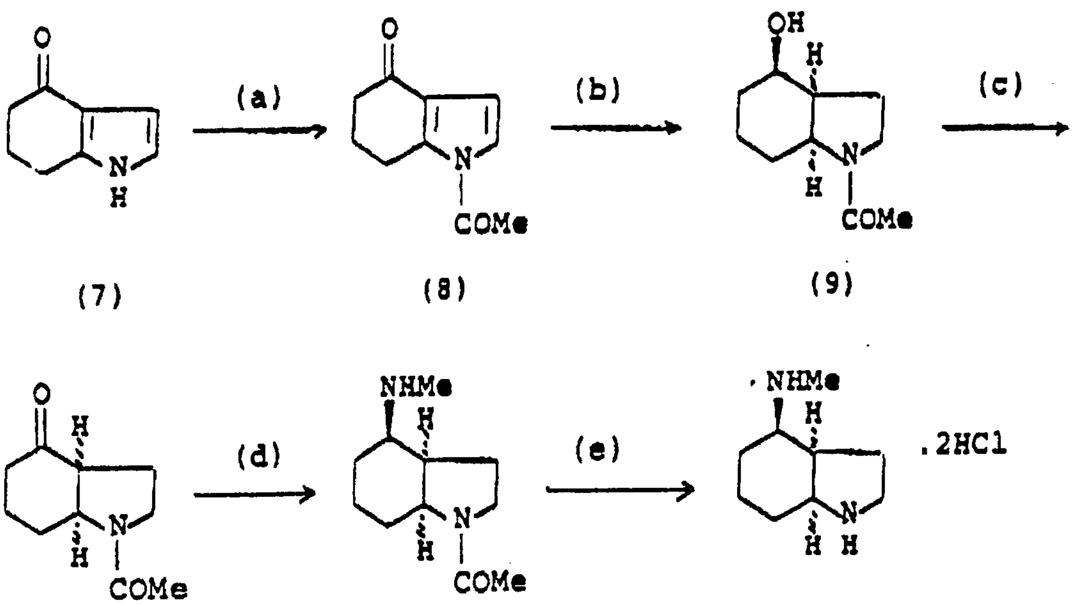


(7) (8) (9)

(10) (11) (III-2)

(a) A mixture of 4-oxo-4,5,6,7-tetrahydroindole (compound (7)) (J.Org.Chem., 43, No. 18, p.354 (1978)) (3.2 g), dry pyridine (15 ml), acetic anhydride (15 ml) and 4-dimethylaminopyridine (360 mg) is stirred at room temperature for 1 hour. The solvent is removed by distillation under reduced pressure, and the residue is combined with methylene chloride, washed with 1N hydrochloric acid and a saturated aqueous sodium chloride solution in order and dried over sodium sulfate. The solvent is removed by distillation, and the residue is chromatographed (eluent: ethyl acetate/n-hexane = 3/2 (v/v)) on silica gel. After removal of the solvent, the residue is combined with a mixture of diethyl ether and n-hexane. The precipitated crystals

are collected by filtration to give the compound (8) (3.8 g).

$^1$H-NMR ($CDCl_3$) δ (ppm): 2.16 (2H, q, J = 6.8 Hz), 2.50 (2H, t, J = 6.8 Hz), 2.58 (3H, s), 3.22 (2H, t, J = 6.8 Hz), 6.63 (1H, d, J = 3.4 Hz), 7.04 (1H, d, J = 3.4 Hz).

(b) To a solution of the compound (8) (4.0 g) in acetic acid (80 ml), platinum dioxide (1.0 g) is added for catalytic reduction under 6 atm. The catalyst is removed by filtration, followed by removal of the solvent under reduced pressure. The residue is combined with potassium carbonate and extracted with ethyl acetate. The solvent is removed from the extract by distillation, and ethyl acetate is added to the residue, from which insoluble materials are removed by filtration. The filtrate is concentrated under reduced pressure, and diethyl ether is added to the residue. Precipitated crystals are collected by filtration to give the compound (9) (1.8 g).

$^1$H-NMR ($CDCl_3$) δ (ppm): 0.94 - 2.18 (8H, m), 2.01 (9/5H, s), 2.07 (6/5H, s), 2.54 (1H, m), 3.37 - 3.64 (2H, m), 3.75 (2/5H, m), 3.97 (1H, m), 4.16 (3/5H, m).

(c) A solution of methylene chloride (200 ml) in dimethylsulfoxide (13.6 ml) is cooled to -78°C, and oxalyl chloride (9.5 ml) is dropwise added thereto while keeping the inner temperature at -60°C. A solution of the compound (9) (10.0 g) in methylene chloride (100 ml) is dropwise added thereto while keeping the inner temperature at -65°C. The reaction mixture is stirred at the same temperature for 30 minutes, and triethylamine (55 ml) is added thereto, followed by stirring to room temperature. The resultant mixture is combined with water, and the organic layer is separated, washed with a saturated aqueous sodium chloride solution and dried over sodium sulfate. The solvent is removed by distillation under reduced pressure, and the residue is chromatographed (eluent: ethyl acetate -methanol/ethyl acetate = 1 : 9 (v/v)) on silica gel. Removal of the solvent from the fractions containing the desired product gives the compound (10) (8.1 g) as an oil.

$^1$H-NMR ($CDCl_3$) δ (ppm): 1.45 - 2.45 (8H, m), 2.04 (2H, s), 2.13 (1H, s), 2.87 (2/3H, m), 2.99 (1/3H, m), 3.39 - 3.70 (2H, m), 4.08 (1/3H, m), 4.41 (2/3H, m).

(d) To a solution of methylamine hydrochloride (4.0 g) in methanol (70 ml), a 20 % methanolic solution of potassium hydroxide (4.5 ml) is added under stirring, and a solution of the compound (10) (7.1 g) in methanol (7 ml) is added thereto. The resultant mixture is stirred at room temperature for 15 minutes, and a solution of sodium cyanoborohydride (5.0 g) in methanol (35 ml) is added thereto, followed by stirring for $3\frac{1}{4}$ hours. To the reaction mixture, a 20 % methanolic solution of potassium hydroxide (15.5 ml) is added, stirring is continued, followed by removal of insoluble materials by filtration. The filtrate is concentrated to approximately 1/3 volume. The concentrate is combined with methylene chloride and a saturated aqueous solution of sodium chloride, stirred and allowed to stand. The organic layer is dried over anhydrous sodium sulfate, and the solvent is removed therefrom. The residue is chromatographed (eluent: methanol/methylene chloride = 5/95 (v/v) - 1 % ammonia-containing methanol/methylene chloride = 10/90 (v/v)) on silica gel. Removal of the solvent from the fractions containing the desired product gives the compound (11) (6.0 g) as an oil.

$^1$H-NMR ($CDCl_3$) δ (ppm): 0.92 - 2.65 (8H, m), 2.00 (9/5H, s), 2.08 (6/5H, s), 2.45 (9/5H, s), 2.46 (6/5H, s), 2.73 (1H, m), 3.35 - 3.65 (2H, m), 3.71 (2/5H, m), 4.14 (3/5H, m).

(e) A solution of the compound (11) (6.0 g) in 2N hydrochloric acid (30 ml) is refluxed for 12 hours under stirring. The reaction mixture is cooled to room temperature, ethyl acetate is added thereto, and the resultant mixture is stirred and allowed to stand. The aqueous layer is separated and concentrated under reduced pressure. The residue is combined with a mixture of ethanol and benzene, followed by removal of the solvent under reduced pressure. The residue is combined with ethanol and ethyl acetate to precipitate crystals, which are collected by filtration to give the compound (III-2) (4.8 g).

$^1$H-NMR ($D_2O$) δ (ppm): 1.34 - 1.56 (3H, m), 1.95 -2.20 (5H, m), 2.76 (3H, s), 2.84 (1H, m), 3.39 - 3.67 (3H, m), 3.94 (1H, m).

Reference Example 3

Preparation of (1R*,6R*,7S*)-7-(N-acetyl-N- methylamino)-3-azabicyclo[4.4.0]decane hydrochoride (III-3):-

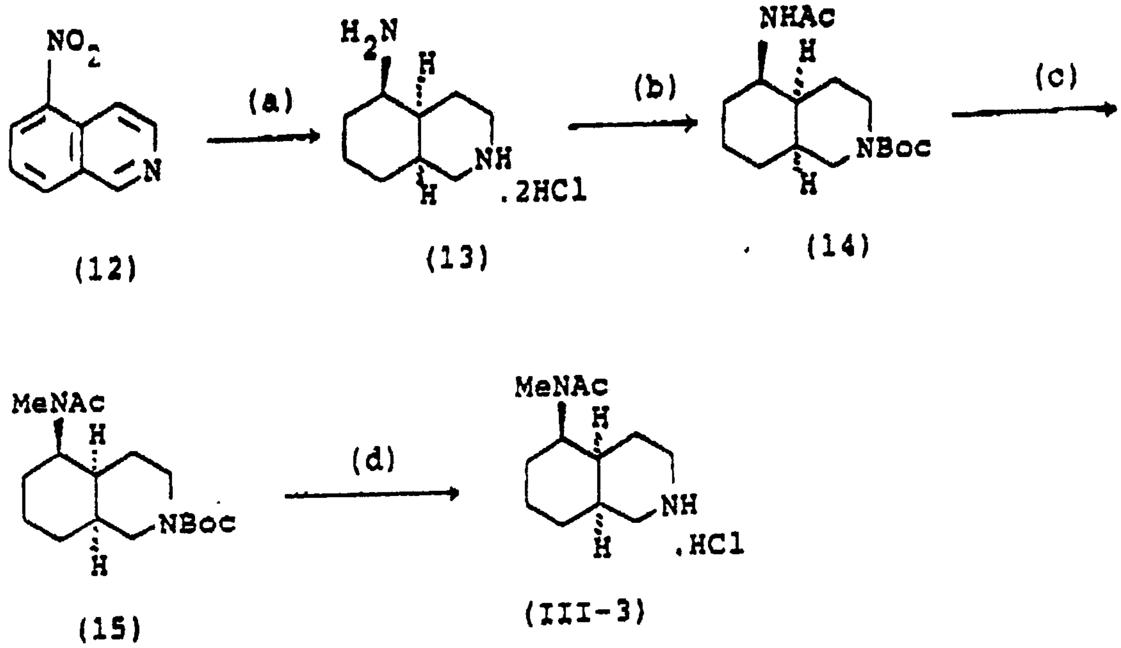


(12) (13) (14) (15) (III-3)

(a) To a solution of 5-nitroisoquinoline (compound (12)) (6.0 g) in acetic acid (120 ml), platinum dioxide (3.0 g) is added, catalytic reduction is carried out under 6 atm. After separation of the catalyst by filtration, the filtrate is concentrated under reduced pressure. Toluene is added to the residue, and the solvent is again removed by distillation under reduced pressure. A 10 % methanolic solution of hydrochloric acid is added to the residue, and the solvent is removed by distillation under reduced pressure. The residue is combined with toluene, followed by concentration under reduced pressure. To the residue, a mixture of methanol and ethyl acetate is added, and precipitate crystals are collected by filtration to give the compound (13) (2.9 g).

$^1$H-NMR ($D_2O$) $\delta$ (ppm): 1.40 - 2.16 (9H, m), 2.35 (1H, m), 3.00 (1H, dt, J = 12.7 Hz, J = 2.9 Hz), 3.19 (1H, dd, J = 12.7 Hz, J = 3.9 Hz), 3.31 (1H, d, J = 12.7 Hz), 3.45 (1H, m), 3.54 (1H, m).

(b) Into a suspension of the compound (13) (3.0 g) in methylene chloride (100 ml), excess gaseous ammonia is introduced, and the thus produced ammonium chloride is removed by filtration, and the filtrate is concentrated under reduced pressure to dryness. The residue is dissolved in a mixture of methanol (6 ml) and water (6 ml) and cooled to 0° C. A solution of di-t-butyl dicarbonate (2.8 g) in methanol (3 ml) is dropwise added thereto, and the resultant mixture is stirred at room temperature overnight, followed by removal of the solvent under reduced pressure. Methylene chloride is added to the residue, which is washed with a saturated aqueous solution of sodium chloride, dried over anhydrous sodium sulfate and concentrated under reduced pressure. The residue is dissolved in pyridine, acetic anhydride is added thereto, and the resultant mixture is stirred at room temperature for 15 minutes. The solvent is removed by distillation under reduced pressure, and the residue is combined with methylene chloride and a saturated aqueous sodium chloride solution, followed by stirring and allowing to stand. The organic layer is dried over anhydrous sodium sulfate and concentrated under reduced pressure. The residue is chromatographed on silica gel to give the compound (14) (1.9 g) as crystals.

$^1$H-NMR ($CDCl_3$) $\delta$ (ppm): 1.21 - 1.82 (9H, m), 1.44 (9H, s), 1.99 (3H, s), 2.16 (1H, m), 2.62 (1H, m), 2.85 (1H, m), 3.97 (2H, m), 4.28 (1H, m), 5.32 (1H, brs).

(c) To a solution of the compound (14) (1.0 g) in dry dimethylformamide (DMF), a 50 % oily dispersion of sodium hydroxide (170 mg) is added, and the mixture is gradually heated until its inner temperature is raised to 75° C. After foaming stops, the reaction mixture is cooled to 20° C under argon stream, and methyl iodide (260 ul) is added thereto, followed by stirring at room temperature overnight. Methylene chloride and water are added to the reaction mixture, which is stirred and allowed to stand. The organic layer is washed with a saturated aqueous solution of sodium chloride and dried over anhydrous sodium sulfate. The solvent is removed by distillation under reduced pressure, and the residue is chromatographed (eluent: ethyl acetate/n-hexane) on silica gel. The fractions containing the desired product are collected, and the solvent is removed by distillation under reduced pressure. A mixture of diethyl ether and n-hexane is added to the residue, the precipitated crystals are removed by filteration, and the filtrate is concentrated to

driness to give the compound (15) (0.9 g).

$^1$H-NMR ($CDCl_3$) δ (ppm): 1.20 - 1.95 (9H, m), 1.44 (9H, s), 2.10 (9/4H, s), 2.15 (3/4H, s), 2.20 (1H, m), 2.57 (1H, m), 2.84 (3/4H, s), 2.85 (1H, m), 2.88 (9/4H, s), 3.68 (1/4H, dt, J = 13.7 Hz, J = 3.5 Hz), 3.91 (1H, m), 4.25 (1H, m), 4.39 (3/4H, dt, J = 13.7 Hz, J = 3.5 Hz).

(d) A solution of the compound (15) (1.3 g) in a 10 % methanolic solution of hydrochloric acid (20 ml) is stirred at 50° C for 15 minutes, followed by addition of the same methanolic solution (10 ml) thereto. Stirring is continued at 50° C for 30 minutes, and the solvent is removed by distillation under reduced pressure. The residue is combined with a mixture of ethanol and ethyl acetate, and the precipitated crystals are collected by filtration to give the compound (III-3) (0.99 g).

$^1$H-NMR ($D_2O$) δ (ppm): 1.40 - 2.32 (10H, m), 2.13 (2H, s), 2.19 (1H, s), 2.84 (1H, s), 2.90 (1H, m), 2.97 (2H, s), 3.11 (1H, m), 3.25 (1H, m), 3.45 (1H, m), 3.92 (1/3H, dt, J = 12.9 Hz, J = 3.7 Hz), 4.25 (2/3H, dt, J = 12.9 Hz, J = 3.7 Hz).

Reference Example 4

Preparation of (1S*,6R*)-2-oxo-8-azabicyclo[4.3.0]nonane hydrochloride (III-4):-

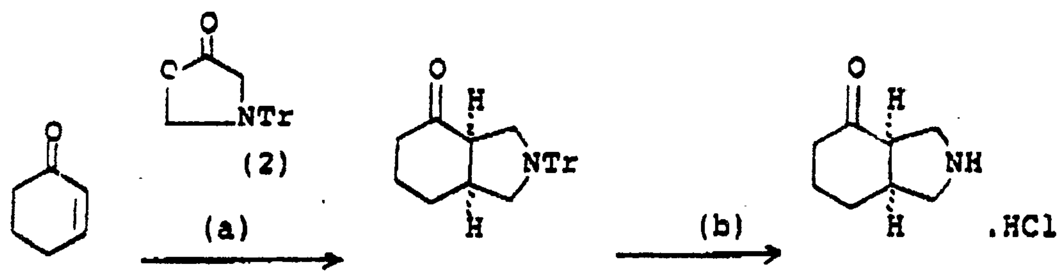

(16) (III-4)

(a) 2-Cyclohexen-1-one (4.5 g) is dissolved in dry toluene (155 ml), and 3-trityl-5-oxazolidinone (2) (15.5 g) is added thereto. The resultant mixture is heated under reflux for 64 hours. After removal of toluene by distillation, the residue is chromatographed (eluent: n-hexane/ethyl acetate = 5/1 (v/v)) on silica gel to give the compound (16) (8.5 g).

IR ($CHCl_3$): 1710, 910 $cm^{-1}$.

$^1$H-NMR ($CDCl_3$) δ (ppm): 1.20 - 2.05 (4H,m);2.20 -2.70 (7H, m), 2.90 - 3.07 (1H, m), 7.08 - 7.53 (15H, m).

(b) A mixture of the compound (16) (5.0 g), trifluroacetic acid (20 ml) and water (20 ml) is stirred at 90° C for 2 minutes. After cooling, trifluroacetic acid and water are removed by distillation under reduced pressure. The residue is combined with ethyl acetate, and the solvent is distilled off. The residue is combined with conc. hydrochloric acid (10 ml) and water (50 ml) and extracted with ethyl acetate. After separation, the aqueous layer is collected and concentrated under reduced pressure. The residue is combined with a mixture of ethanol and ethyl acetate, and the precipitated crystals are collected by filtration to give the compound (III-4) (2.1 g).

$^1$H-NMR ($D_2O$) δ (ppm): 1.68 - 1.92 (2H, m), 1.97 -2.15 (2H, m), 2.38 - 2.63 (2H, m), 2.89 (1H, m), 3.05 (1H, m), 3.22 - 3.43 (3H, m), 3.89 (1H, m).

Reference Example 5

Preparation of (1R*,2S*,6S*)-2-methylamino-7-azabicyclo[4.3.0]nonane hydrochloride (III-5):-

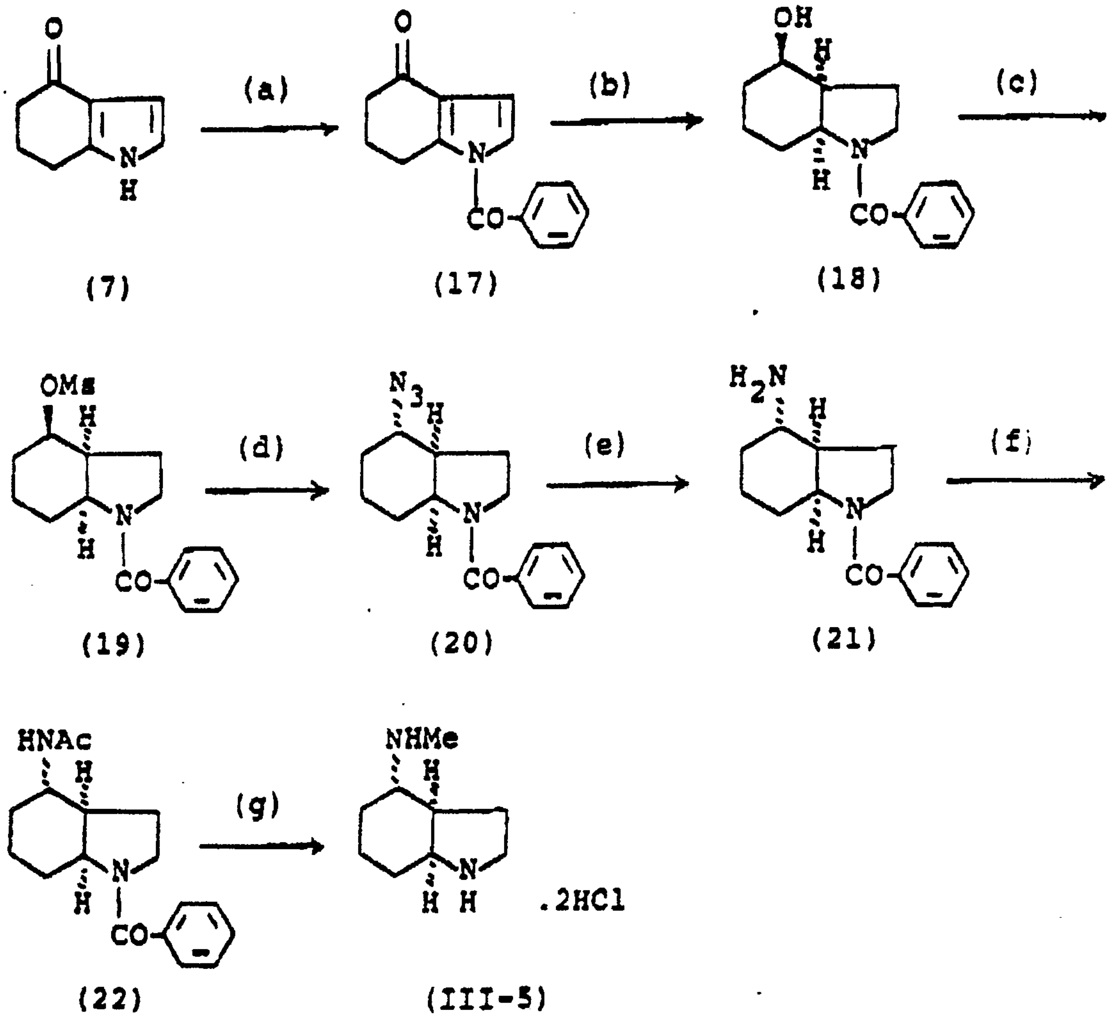


(7) (17) (18) (19) (20) (21) (22) (III-5)

(a) To a solution of 4-oxo-4,5,6,7-tetrahydroindole (compound (7)) (5 g) in dimethylformamide (25 ml), sodium hydride (60 % oily dispersion) (1.8 g) is added under ice-cooling, followed by stirring for 5 minutes. Benzoyl chloride (62 g) is dropwise added thereto, and the resultant mixture is stirred at room temperature for 10 minutes. The reaction mixture is poured into ice-water and extracted with ethyl acetate. The extract is washed with water, dried over sodium sulfate and concentrated by distillation under reduced pressure. The residue is washed with a mixture of ethyl acetate and isopropyl ether to give the compound (17) (6.5 g; yield, 73 %) as crystals. m.p., 121 - 122° C.

IR (nujol): 1690, 1650 $cm^{-1}$.

$^1$H-NMR ($CDCl_3$) $\delta$ (ppm): 1.90 - 2.60 (4H, m), 3.15 - 3.27 (2H, m), 6.58 (1H, d, J = 4.5 Hz), 6,87 (1H, d, J = 4.5 Hz), 7.40 - 7.80 (5H, m).

(b) To a solution of the compound (17) (5 g) in acetic acid (50 ml), platinum dioxide monohydrate (1 g) is added, and catalytic reduction is carried out under 5 atm. The catalyst is removed by filtration, and acetic acid is removed by distillation under reduced pressure. The residue is combined with sodium hydrogencarbonate and extracted with methylene chloride. The extract is washed with water, dried over anhydrous sodium sulfate and concentrated under reduced pressure. The residue is chromatographed (eluent: ethyl acetate - methanol/ethyl acetate = 5/95 (v/v)) on silica gel. Removal of the solvent from the fractions containing the desired product gives the compound (18) (6.3 g) as an oil.

IR (film): 3370, 1650, 1435, 790 $cm^{-1}$.

$^1$H-NMR ($CDCl_3$) $\delta$ (ppm): 0.90 - 2.70 (9H, m), 3.20 - 4.50 (5H, m), 7.30 - 7.50 (5H, m).

(c) To a solution of the compound (18) (5.5 g) in dry methylene chloride (40 ml), triethylamine (3.3 g) is added. After addition of methanesulfonyl chloride (3.76 g) thereto under ice-cooling, the reaction is carried out for 15 minutes. The reaction mixture is combined with an aqueous solution of sodium hydrogencarbonate and extracted with methylene chloride. The extract is washed with water, dried over anhydrous

sodium sulfate and concentrated under reduced pressure. The residue is chromatographed (eluent: methylene chloride - methanol/methylene chloride = 3/97 (v/v)) on silica gel to give the compound (19) (5.9 g) as an oil.

IR (film): 1520, 1420, 1345, 1170, 790 $cm^{-1}$.

$^1$H-NMR ($CDCl_3$) δ (ppm): 0.90 - 2.40 (9H, m), 2.60 - 2.90 (1H, m), 3.04 (3H, s), 3.30 - 3.80 (2H, m), 4.80 -5.05 (1H, m), 7.35 - 7.50 (5H, m).

(d) To a solution of the compound (19) (5.9 g) in dimethylformamide (59 ml), water (5.9 ml) and sodium azide (1.75 g) are added, and the resultant mixture is allowed to react at 120 °C for 20 minutes. The solvent is removed by distillation, and the residue is combined with ice-water and extracted with ether. The extract is washed with water, dried over anhydrous sodium sulfate and concentrated under reduced pressure. The residue is chromatographed (eluent: ethyl acetate/n-hexane = 1/2 (v/v) - ethyl acetate/n-hexane = 1/1 (v/v)) to give the compound (20) (1.3 g) as an oil.

IR (film): 2075, 1620, 1410, 780 $cm^{-1}$.

$^1$H-NMR ($CDCl_3$) δ (ppm): 1.20 - 2.40 (9H, m), 3.30 - 4.00 (4H, m), 7.30 - 7.50 (5H, m).

(e) To a solution of the compound (20) (1.3 g) in methanol (30 ml), 10 % palladium-carbon (800 mg) is added, and catalytic reduction is carried out. The catalyst is removed by filtration, and the filtrate is concentrated under reduced pressure to give the compound (21) as an oil.

(f) To the compound (21) as above obtained, acetic anhydride (20 ml) is added, followed by heating on a water batah for 10 minutes. Acetic anhydride is removed by distillation, and sodium hydrogencarbonate is added thereto. The resultant mixture is extracted with methylene chloride, and the extract is washed with water and dried over anhydrous sodium sulfate. After removal of the solvent, the residue is chromatographed (eluent: methanol/ethyl acetate = 1/9 (v/v)) on silica gel to give the compound (22) (540 mg) as an oil.

IR ($CHCl_3$): 1415, 1615, 1665 $cm^{-1}$.

$^1$H-NMR ($CDCl_3$) δ (ppm): 1.33 - 2.20 (9H, m), 1.94 (3H, s), 3.60 - 3.78 (1H, m), 4.04 - 4.20 (2H, m), 4.40 (1H, brs), 6.01 (1H, brs), 7.35 - 7.59 (5H, m).

(g) To a solution of the compound (22) (2 g) in dry dimethylformamide (40 ml), sodium hydride (60 % oily dispersion) (560 mg) is added, and the resultant mixture is stirred at 60 to 70 °C for 90 minutes. The reaction mixture is cooled to room temperature, methyl iodide (1.99 g) is dropwise added thereto, and the resultant mixture is stirred overnight. Ice-water is added to the reaction mixture, which is extracted with methylene chloride. The extract is washed with water, dried over anhydrous sodium sulfate and concentrated under reduced pressure. The residue is chromatographed (eluent: methanol/ethyl acetate = 5/95 (v/v) - and methanol/ethyl acetate = 7/93 (v/v)) on silica gel. The fractions containing the desired product are collected, and the solvent is removed by distillation to give the N-methyl compound (0.80 g) as an oil.

To the oil (0.80 g), conc. hydrochloric acid (30 ml) is added, and the resultant mixture is stirred at 130 °C for 11 hours under reflux. After cooling, the reaction mixture is combined with ethyl acetate and water, stirred and allowed to stand. The aqueous layer is separated and concentrated under reduced pressure, and sodium carbonate is added to the residue, which is extracted with a mixture of metanol and methylene chloride. The extract is concentrated under reduced pressure to driness, and acetonitrile is added thereto. The precipitated crystals are collected by filtration, and the filtrate is concentrated to give the compound (III-5) (0.25 g) as an oil.

$^1$H-NMR ($D_2O$) δ (ppm): 1.18 - 2.00 (9H, m), 2.11 (3H, s), 2.19 - 2.29 (2H, m), 2.83 - 2.95 (2H, m).

Reference Example 6

Preparation of (1R*,2S*,6S*)-2-methylamino-8-azabicyclo[4.3.0]nonane hydrochloride (III-6):-

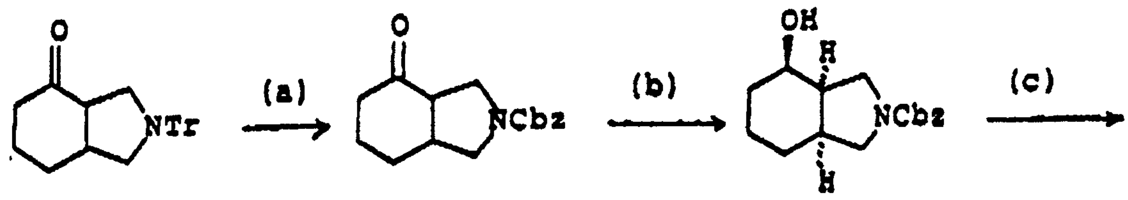


(23) (24) (25)

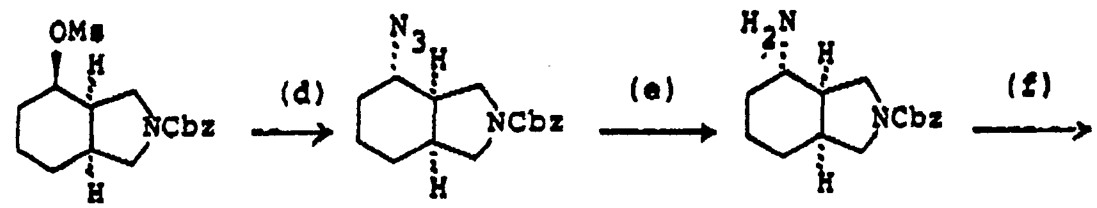


(26) (27) (29)

+

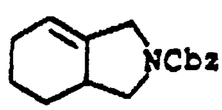

(28)

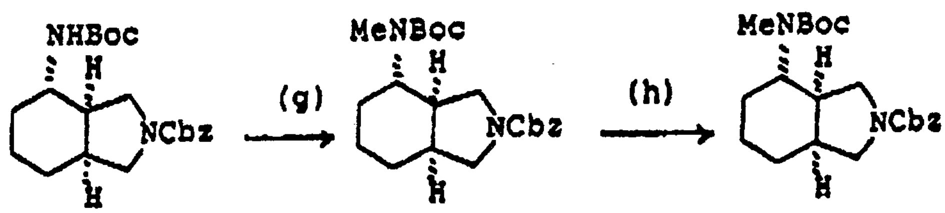


(30) (31) (III-6)

(a) To a solution of the compound (23) (35 g; 9.17 mmol) in trifluoroacetic acid (20 ml), water (20 ml) is added, and the resultant solution is stirred at room temperature for 5 minutes. Excess water is added to the reaction mixture, and the aqueous layer is washed with ethyl acetate. To the washed aqueous layer, sodium carboate (30 g, 284 mmol) and benzyl chlorocarbonate (1.72 g, 10.1 mmol) are added, and the resultant mixture is stirred at room temperature for 50 minutes. The reaction mixture is extracted with ether, and the ether extract is washed with water, dried over magnesium sulfate and concentrated by distillation under reduced pressure to give the compound (24) (1.67 g; yield, 67 %) as a crude product.

$^1$H-NMR ($CDCl_3$) $\delta$ (ppm): 1.58 - 1.82 (2H, m), 1.82 - 2.02 (2H, m), 2.24 - 2.52 (2H, m), 2.73 - 2.95 (2H, m), 3.11 - 3.26 (1H, m), 3.31 - 3.57 (2H, m), 3.93 - 4.06 (1H, m), 5.03 - 5.21 (2H, m), 7.26 - 7.48 (15H, m).

(b) A solution of the compound (24) (1.67 g, 6.1 mmol) in dry tetrahydrofuran (17 ml) is cooled to -78° C, and L-Selectride[R] (lithium tri-sec-butylborohydride, 1.0 M solution in tetrahydrofuran (9.2 ml, 9.2 mmol) is added thereto below -70° C. The resultant mixture is stirred at -78° C for 30 minutes and further at room temperature for 2 hours. Under ice-cooling, water (1 ml) and 30 % aqueous hydrogen peroxide (4 ml) are added to the reaction mixture at a temperature below 30° C, followed by stirring at room temperature for 30 minutes. The reaction mixture is poured into ice-water and extracted with ether. The ether extract is washed with water, dried over magnesium sulfate and concentrated under reduced pressure. The residue is

chromatographed (eluent: toluene/ethyl acetate = 2/13 (v/v)) on silica gel to give the compound (25) (1.40 g; yield, 83 %).

$^1$H-NMR ($CDCl_3$) δ (ppm): 1.02 - 1.74 (7H, m), 2.06 - 2.26 (1H, m), 2.51 - 2.72 (1H, m), 3.28 - 3.63 (4H, m), 3.90 - 4.05 (1H, m), 5.04 - 5.23 (2H, m), 7.44 (5H, m).

(c) To a solution of the compound (25) (1.40 g, 51 mmol) and triethylamine (617 mg, 6.1 mmol) in methylene chloride (28 ml), methanesulfonyl chloride (641 mg, 5.6 mmol) is added under ice-cooling, followed by stirring at room temperature for 40 minutes. The reaction mixture is washed with aqueous sodium hydrogencarbonate and water in order, and the methylene chloride layer is dried over sodium sulfate and concentrated under reduced pressure to give the compound (26) (1.75 g; yield, 97 %) as crystals. m.p., 100 - 104° C.

$^1$H-NMR ($CDCl_3$) δ (ppm): 1.05 - 2.00 (6H, m), 2.20 - 2.30 (1H, m), 2.75 - 2.90 (1H, m), 2.99 (3H, s), 3.30 -3.65 (4H, m), 4.90 - 5.08 (1H, m), 5.14 (2H, s), 7.03 - 7.40 (5H, m).

(d) A mixture of the compound (26) (1.4 g, 5.9 mmol), sodium azide (773 mg, 11.9 mmol), dimethylformamide (20 ml) and water (2 ml) is stirred at 120° C for 1 hour and poured into ice-water, followed by extraction with ether. The ether extract is washed with water, dried over magnesium sulfate and concentrated under reduced pressure. The residue is chromatographed (eluent: toluene/ethyl acetate = (v/v)) on silica gel to give a mixture of the compound (27) (608 mg, yield, 51 %) and the compound (28) (359 mg; yield, 35 %).

Compound (27)

IR (neat): 2090, 1695, 1410 $cm^{-1}$.

$^1$H-NMR ($CDCl_3$) δ (ppm): 1.40 - 1.70 (5H, m), 1.90 - 2.10 (2H, m), 2.40 - 2.55 (1H, m), 3.15 - 3.65 (5H, m), 5.09 (1H, d, J = 10 Hz), 5.18 (1H, d, J = 10 Hz), 7.30 -7.40 (5H, m).

(e) A mixture of the compound (27) (608 mg, 2.02 mmol), triphenylphosphine (637 mg, 24 mmol), tetrahydrofuran (30 ml) and water (3 ml) is stirred at 60° C for 4 hours, and then the solvent is removed by distillation under reduced pressure. Dilute hydrochloric acid is added to the reaction mixture, which is washed with ether, made alkaline with potassium carbonate and extracted with ether. The ether extract is washed with water, dried over magnesium sulfate and concentrated under reduced pressure to give the compound (29) (444 mg; yield, 80 %).

$^1$H-NMR ($CDCl_3$) δ (ppm): 1.03 - 1.78 (8H, m), 2.32 - 2.45 (2H, m), 3.12 - 3.69 (5H, m), 5.11 (1H, d, J = 13 Hz), 5.15 (1H, d, J = 13 Hz), 7.24 - 7.74 (5H, m).

(f) To a solution of the compound (29) (440 mg, 1.6 mmol) in methylene chloride (20 ml), di-t-butyl dicarbonate (422 mg, 1.76 mmol) is added, and the resultant mixture is allowed to stand at room temperature for 15 hours. The reaction mixture is then chromatographed (eluent: toluene/ethyl acetate = 4/1 (v/v)) on silica gel to give the compound (30) (387 mg; yield, 64 %).

$^1$H-NMR ($CDCl_3$) δ (ppm): 1.09 - 1.38 (1H, m), 1.44 (9H, s), 1.48 - 1.68 (4H, m), 1.80 - 2.06 (2H, m), 2.30 -2.54 (1H, m), 3.18 - 3.63 (5H, m), 4.30 - 4.48 (1H, m), 5.02 - 5.23 (2H, m), 7.15 - 7.43 (5H, m).

(g) To a solution of the compound (30) (380 mg, 1.0 mmol) in dimethylformamide (10 ml), sodium hydride (60 % oil dispersion) (45 mg, 1.1 mmol) and methyl iodide (158 mg, 1.1 mmol) are added, and the resultant mixture is stirred at 60° C for 1 hour. The reaction mixture is poured into ice-water and extracted with ether. The ether extract is washed with water, dried over magnesium sulfate and concentrated under reduced pressure. The residue is chromatographed (eluent: toluene/ethyl acetate = 1/1 (v/v)) on silica gel to give the compound (31) (381 mg; yield, 97 %).

$^1$H-NMR ($CDCl_3$) δ (ppm): 1.27 - 1.78 (15H, m), 2.02 - 2.28 (1H, m). 2.46 - 2.70 (4H, m), 3.17 - 3.58 (4H, m), 3.67 - 4.00 (1H, m), 4.99 - 5.27 (2H, m), 7.13 - 7.57 (5H, m).

(h) To a solution of the compound (31) (375 mg, 0.97 mmol) in methanol (30 ml), 10 % palladium-carbon (200 mg) is added, and catalytic reduction is carried out at room temperature under atmospheric pressure. The catalyst is removed by filtration. Removal of the solvent from the filtrate under reduced pressure gives the compound (III-6) (226 mg; yield, 92 %) as a crude product.

$^1$H-NMR ($CDCl_3$) δ (ppm): 1.22 - 1.74 (14H, m), 1.87 - 2.60 (4H, m), 2.67 - 3.01 (7H, m), 3.86 - 4.06 (1H, m).

Example 1

Preparation of 7-[(1R*,5S*)-1-methylaminomethyl-3-azabicyclo[3.3.0]octan-3-yl]-1-cyclopropyl-6-fluoro-1,4-dihydro-4-oxo-3-quinolinecarboxylic acid (I-1):-

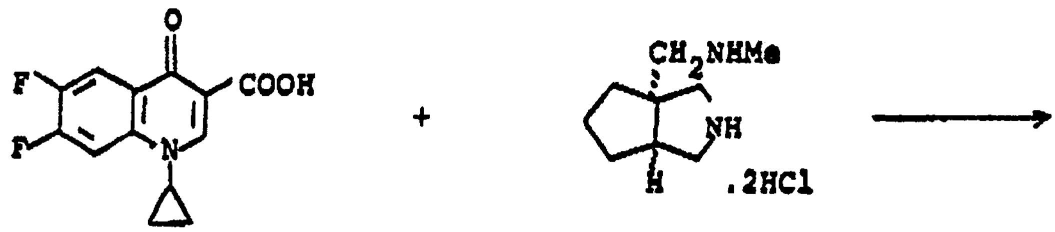

(II-1) (III-1)

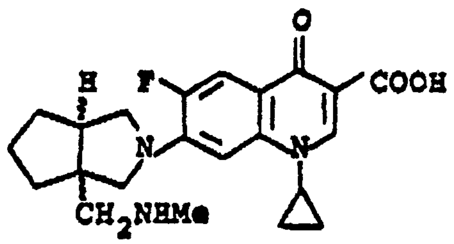

(I-1)

To a suspension of 1-cyclopropyl-6,7-difluoro-1,4-dihydro-4-oxo-3-quinolinecarboxylic acid (II-1) (500 mg) and (1R*,5S*)-1-methylaminomethyl-3-azabycyclo[3.3.0]octane hydrochloride (III-1) (720 mg) in acetonitrile (10 ml), 1,8-diazabicyclo[5.4.0]undec-7-ene (DBU) (1.3 ml) is added under stirring, and the resultant mixture is heated under reflux for 2 hours. After cooling, the precipitated crystals are collected by filtration and recrystallized from a mixture of ethanol and chloroform to give the compound (I-1) (530 mg; yield, 61 %). m.p., 229 - 231 °C.

| Elementary analysis (%) for $C_{22}H_{26}FN_3O_3.1/5C_2H_5OH.\ 1/2CO_2.3/2H_2O$: | | | | |
|---|---|---|---|---|
| Calcd.: | C, 60.10; | H, 6.61; | F, 4.16; | N, 9.19. |
| Found: | C, 60.21; | H, 6.33; | F, 4.24; | N, 9.12. |

Examples 2 to 6

In the same manner as in Examples 1, the pyridone-carboxylic acid compounds (I) as shown in Table 1 are produced.

**Table 1**

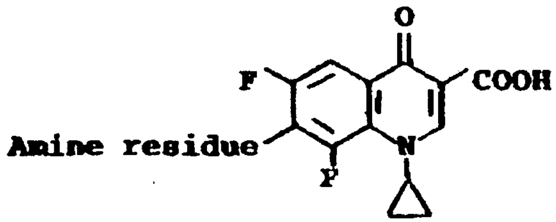

(I)

| Example No. | Compound No. | Amine residue | Melting point (°C) | Molecular formula | Configuration |
|---|---|---|---|---|---|
| 2 | I-2 | 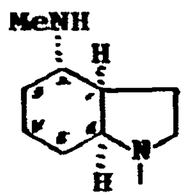 | 263-267 (decomp.) | $C_{22}H_{25}F_2N_3O_3 \cdot H_2O$ | 1R*,2S*,6S* |
| 3 | I-3 | 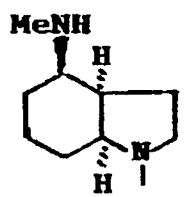 | 267-270 (decomp.) | $C_{22}H_{25}F_2N_3O_3 \cdot 1/2CO_2 \cdot 2H_2O$ | 1R*,2R*,6S* |

(Continued)

| | | | | | |
|---|---|---|---|---|---|
| 4 | I-4 | 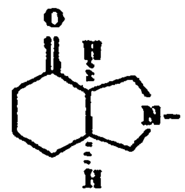 | 235-236 | $C_{21}H_{20}F_2N_2O_4$ $.1/4CO_2.1/4H_2O$ | 1S*,6R* |
| 5 | I-5 | 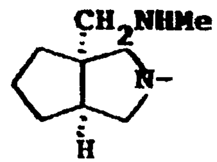 | 270-271 (decomp.) | $C_{22}H_{25}F_2N_3O_3$ $.HCl.1/2H_2O$ | 1R*,5S* |
| 6 | I-6 | 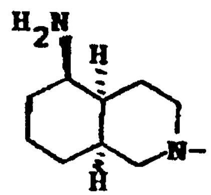 | 215-217 (decomp.) | $C_{22}H_{25}F_2N_3O_3$ $.2.7H_2O$ | 1R*,6R*,7S* |

Example 7

Preparation of 1-cyclopropyl-6,8-difluoro-7-[(1R*,6R*,7S*)-7-methylamino-3-azabicyclo[4.4.0]decan-3-yl]-1,4-dihydro-4-oxo-3-quinolinecarboxylic acid hydrochloride (I-7):-

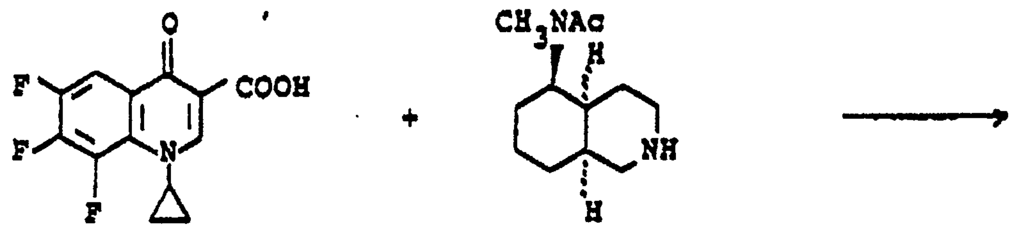

(II-2) (III-3)

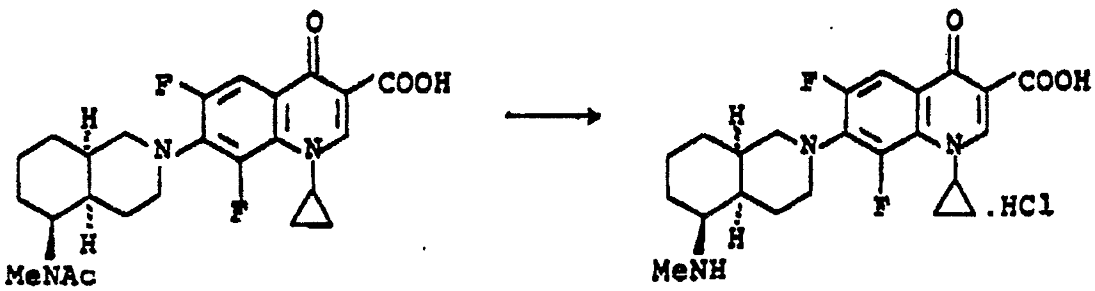

(I-5a) (I-7)

To a suspension of (1R*,6R*,7S*)-7-(N-acetyl-N-methylamino)-3-azabicyclo[4.4.0]decane hydrochloride (III-3) (900 mg) and 1-cyclopropyl-6,7,8-trifluoro-1,4-dihydro-4-oxo-3-quinoline carboxylic acid (II-2) (1.0 g) in acetonitrile (10 ml), DBU (1.2 g) is added under stirring, and the resultant mixture is heated under reflux for 12 hours. Acetonitrile is removed by distillation under reduced pressure, and the residue is dissolved in methylene chloride. The resultant solution is washed with 1N hydrochloric acid and a saturated aqueous solution of sodium chloride, dried over anhydrous sodium sulfate and concentrated. The residue is crystallized from a mixture of ethyl acetate and ether and recrystallized from a mixture of methylene chloride and ethanol to give the compound (I-7a) (860 mg).

The thus obtained compound (I-7a) is combined with 6N hydrochloric acid (17 ml) and refluxed overnight. After removal of the solvent, the residue is combined with a mixture of ethanol and toluene, and the solvent is removed by distillation. The residue is crystallized from a mixture of ethanol and ethyl acetate, and the precipitated crystals are collected by filtration and recrystallized from ethanol to give the compound (I-7) (93 mg) as crystals. m.p., 280 -282 °C (decomp.).

| Elementary analysis (%) for $C_{23}H_{28}ClF_2N_3O_3$: | | | | |
|---|---|---|---|---|
| Calcd.: | C, 59.03; | H, 6.03; | N, 8.98; | F, 8.12. |
| Found: | C, 58.91; | H, 6.16; | N, 8.93; | F, 7.86. |

Example 8

Preparation of 7-[(1R*,5S*)-1-aminomethyl-3-azabicyclo[3.3.0]octan-3-yl]-1-cyclopropyl-6-fluoro-1,4-dihydro-4-oxo-3-quinolinecarboxylic acid (I-8):-

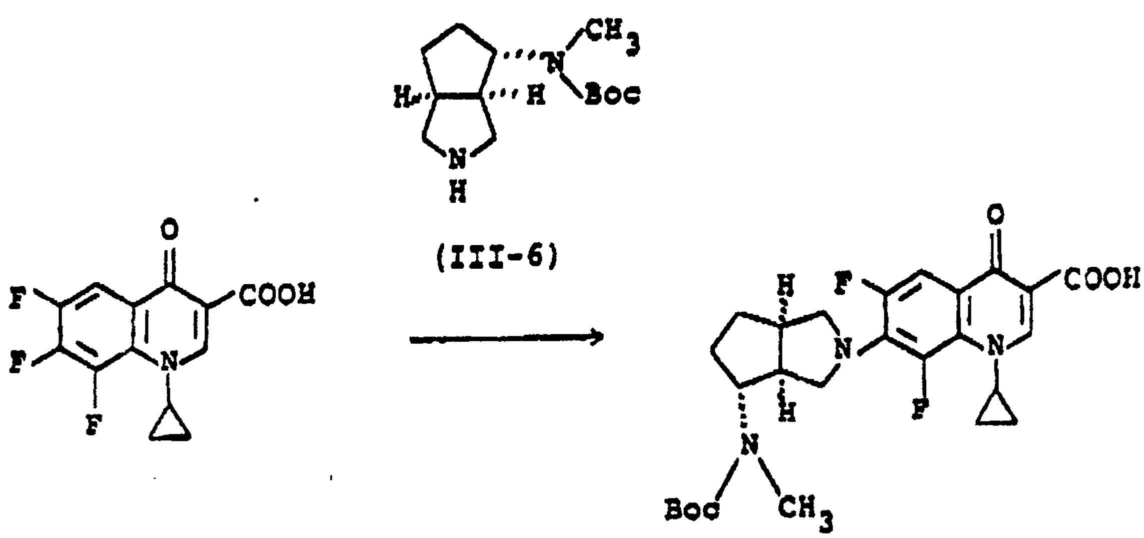

(III-6)

(II-2) (I-8a)

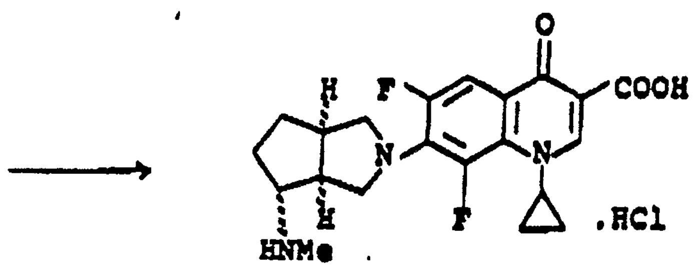

(I-8)

A mixture of quinoline carboxylic acid (II-2) (163 mg, 0.58 mmol), (1R*,2S*,6S*)-2-methylamino-8-azabicyclo-[4.3.0]nonane (III-6) (220 mg, 0,86 mmol), DBU (145 mg, 0.95 mmol) and acetonitrile (4 ml) is heated under reflux for 2 hours, followed by removal of the solvent. The residue is chromatographed (eluent: methanol/methylene chloride = 5/95 (v/v)) on silica gel. The fractions containing the desired product are collected and concentrated under reduced pressure. The residue is washed with isopropyl ether to give the compound (I-8a) (yield, 82 %).

The thus obtained compound (I-8a) (235 mg, 0.45 mmol) is added to a methanolic solution of hydrochloric acid, and the resultant mixture is stirred at 80 °C for 5 minutes. The solvent is removed by distillation from the reaction mixture under reduced pressure, and the residue is recrystallized from a mixture of methanol and ethanol to give the compound (I-8) (182 mg). m.p., 290 °C (decomp.).

Elementary analysis (%) for $C_{22}H_{26}ClFN_3O_3.1/3H_2O$:

Calcd.: C, 57.45; H, 5.84; Cl, 7.71; F, 8.26: N, 9.14.

Found: C, 57.46; H, 5.72; Cl, 8.10; F, 8.24; N, 9.15.

Example 9

Preparation of 7-[(1R*,5S*)-1-methylaminomethyl-3-azabicyclo[3.3.0]octan-3-yl]-1-cyclopropyl-6-chloro-1,4-dihydro-4-oxo-3-quinoline carboxylic acid (I-9):-

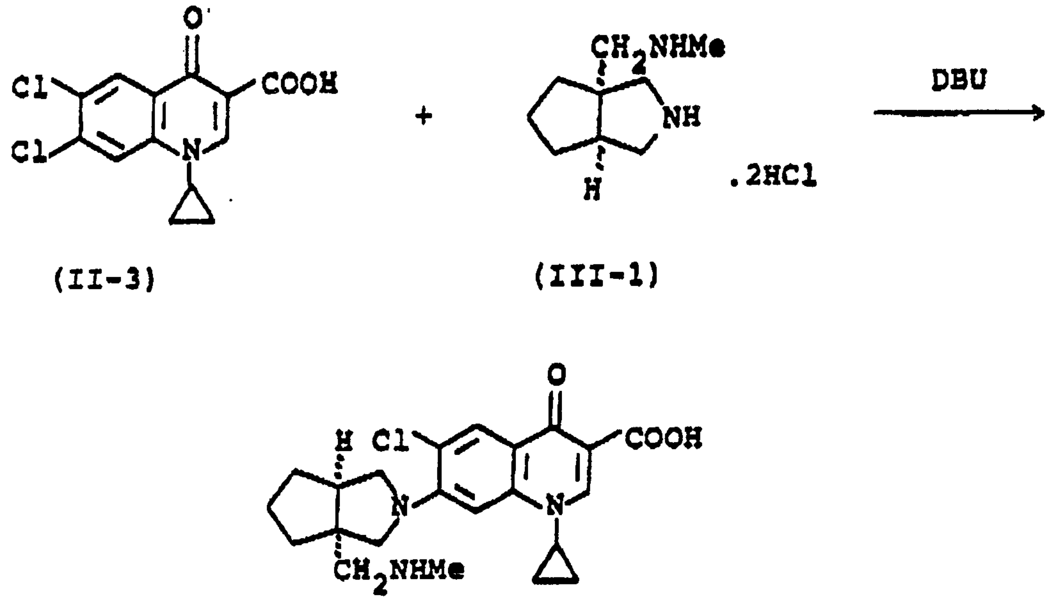


(II-3) (III-1)

(I-9)

To a suspension of 1-cyclopropyl-6,7-dichloro-1,4-dihydro-4-oxo-3-quinoline carboxylic acid (II-3) (6.1 g) and (1R*,5S*)-1-methylaminomethyl-3-azabicyclo[3.3.0]octane hydrochloride (III-1) (7.0 g) in acetonitrile (61 ml), DBU (13 ml) is added under stirring, and the resultant mixture is heated under reflux for 64 hours. After cooling, the precipitated crystals are collected by filtration and washed with acetonitrile to give the objective compound (I-9) (5.8 g; yield, 68 %).

$^1$H-NMR ($CDCl_3$) $\delta$ (ppm): 1.16 - 1.86 (10H, m), 2.58 (1H, m), 2.49 (3H, s), 2.67 (2H, s), 3.29 (1H, dd, J = 10.1 Hz, 4.9 Hz), 3.36 (1H, d, J = 9.9 Hz, 3.50 (1H, m), 3.60 (1H, d, J = 9.9 Hz), 3.73 (1H, dd, J = 10.1 Hz, 7.9 Hz), 7.21 (1H, s), 8.37 (1H, s), 8.76 (1H, s).

Example 10

Preparation of 7-[(1R*,5S*)-1-methyltrifluoroacetylaminomethyl-3-azabicyclo[3.3.0]octan-3-yl]-1-cyclopropyl-6,8-dichloro-1,4-dihydro-4-oxo-3-quinolinecarboxylic acid (I-9a):-

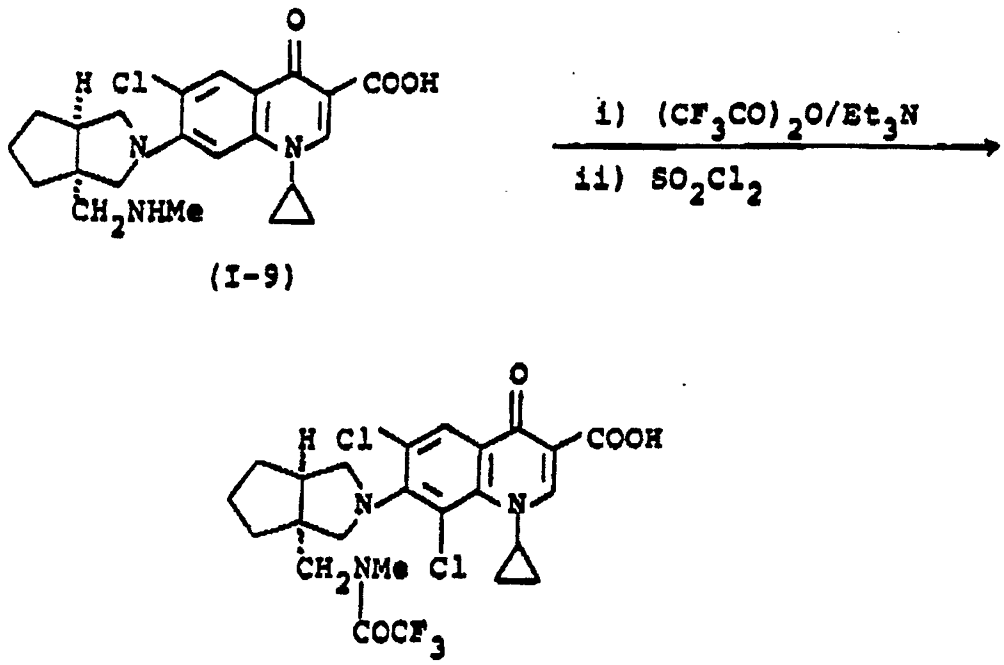


(I-9)

(I-9a)

To a suspension of 7-[(1R*,5S*)-1-methylaminomethyl-3-azabicyclo[3.3.0]octan-3-yl)-1-cyclopropyl-6-chloro-1,4-dihydro-4-oxo-3-quinoline carboxylic aid (I-9) (1.0 g) in dichloromethane (10 ml), triethylamine (0.5 ml) and trifluoroacetic anhydride (0.5 ml) are added, and the resultant mixture is stirred at room temperature for 45 minutes. The reaction mixture is cooled to 0° C, and a 1M dichloromethane solution of sulfuryl chloride (3.8 ml) is dropwise added thereto, followed by stirring for 5 minutes. The reaction mixture is poured into ice-water and vigorously stirred. The lower layer is separated, washed with water and a saturated aqueous solution of sodium chloride in order and dried over anhydrous magnesium sulfate. After removal of the solvent, the residue is chromatographed (eluent: ethyl acetate) on silica gel to give the compound (I-9a) (270 mg; yield, 21 %).

$^1$H-NMR ($CDCl_3$) $\delta$ (ppm): 0.88 - 1.88 (10H, m), 2.54 (1H, m), 3.07 (1H, dd, J = 9.8 Hz, 5.2 Hz), 3.22 (3H, brs), 3.40 (1H, d, J = 9.8 Hz), 3.48 (1H, d, J = 9.8 Hz), 3.58 (1H, d, J = 13.9 Hz), 3.82 (1H, d, J = 13.9 Hz), 4.07 (1H, dd, J = 9.8 Hz, 8.2 Hz), 4.30 (1H, m), 8.36 (1H, s), 8.91 (1H, s).

Example 11

Preparation of 7-[(1R*,5S*)-1-methylaminomethyl-3-azabicyclo[3.3.0]octan-3-yl]-1-cyclopropyl-6,8-dichloro-1,4-dihydro-4-oxo-3-quinoline carboxylic acid hydrochloride (I-10):-

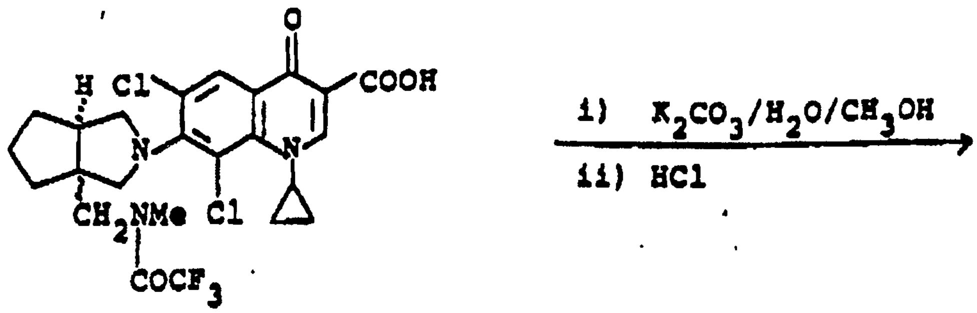


(I-9a)

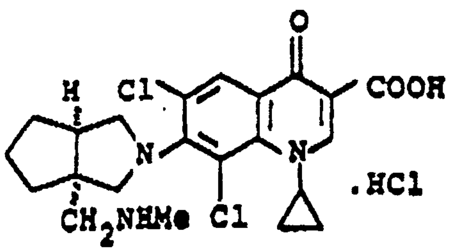

(I-10)

7-[(1R*,5S*)-1-Methyltrifluoroacetylaminomethyl-3-azabicyclo[3.3.0]octan-3-yl]-1-cyclopropyl-6-fluoro-1,4-dihydro-4-oxo-3-quinolinecarboxylic acid (I-9a) (270 mg) and potassium carbonate (200 mg) are dissolved in a mixture of water (14 ml) and methanol (7 ml), and the resultant solution is stirred at room temperature for 45 minutes and further at 50 °C for 2 hours. The reaction mixture is concentrated by distillation under reduced pressure, adjusted to pH 8 with 1N hydrochloric acid and further adjusted to pH 7 with acetic acid. The precipitated crystals are collected by filtration and washed with water and ethanol. The crystals thus obtained are suspended in ethanol (10 ml), 1N hydrochloric acid (10 ml) is added thereto to make a solution, and the solvent is removed by distillation. The residue is dissolved in ethanol, diethyl ether is added thereto, and the precipitated crystals are collected by filtration to give th compound (I-10) (120 mg; yield, 50 %). m.p., 220 °C (decomp.) (recrystallized from aqueous ethanol).

| Elementary analysis (%) for $C_{22}H_{26}Cl_3N_3O_3$. 79/50$H_2O$: | | | | |
|---|---|---|---|---|
| Calcd.: | C, 51.28; | H, 5.70; | Cl, 20.64; | N, 8.15. |
| Found: | C, 51.56; | H, 5.46; | Cl, 20.38; | N, 8.42. |

## Example 12

Preparation of 7-[(1R*,5S*)-1-(N-methyl-N-trifluoroacetylaminomethyl)-3-azabicyclo[3.3.0]octan-3-yl]-1-cyclopropyl-8-chloro-6-fluoro-1,4-dihydro-4-oxoquinolinecarboxylic acid (I-Ia):-

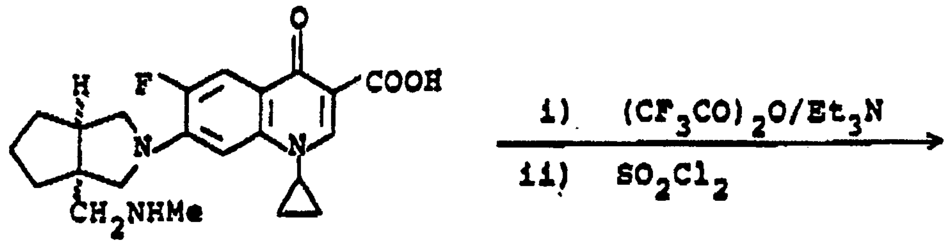


(I-1)

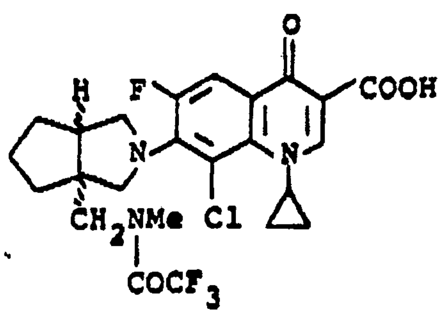

(I-1a)

To a suspension of 7-[(1R*,5S*)-1-methylaminomethyl-3-azabicyclo[3.3.0]octan-3-yl]-1-cyclopropyl-6-fluoro-1,4-dihydro-4-oxo-3-quinolinecarboxylic acid (I-1) (1.0 g) in dichloromethane (50 ml), triethylamine (0.72 ml) is added, and the resultant mixture is cooled to -78° C. A solution of trifluoroacetic anhydride (0.71 ml) in dichloromethane (5 ml) is dropwise added thereto. The resulting mixture is gradually heated to room temperature, followed by stirring at the same temperature for 30 minutes. The reaction mixture is again cooled to -78° C, and a solution of sulfuryl chloride (0.2 ml) in dichloromethane (5 ml) is dropwise added thereto, heated to room temperature and stirred for 10 minutes. The reaction mixture is poured into ice-water and vigorously stirred. The lower layer is separated, washed with water and a saturated aqueous sodium chloride solution in order and dried over magnesium sulfate. After removal of the solvent, the residue is purified by TLC (developing solvent: ethyl acetate/hexane = 2/1 (v/v); eluent: ethyl acetate) to give the compound (I-1a) (360 mg; yield, 27 %).
$^1$H-NMR ($CDCl_3$) $\delta$ (ppm): 0.91 - 1.89 (10H, m), 2.50 (1H, m), 3.20 (1H, m), 3.22 (3H, bs), 3.50 (2H, bs), 3.66 (2H, bs), 3.98 (1H, t, J = 8.5 Hz), 4.31 (1H, m), 8.00 (1H, d, J = 12.5 Hz), 8.91 (1H, s), 14.52 (1H, s).

Example 13

Preparation of 7-[(1R*,5S*)-1-methylaminomethyl-3- azabicyclo[3.3.0]octan-3-yl]-1-cyclopropyl-8-chloro-6-fluoro-1,4-dihydro-4-oxo-3-quinolinecarboxylic acid hydrochloride (I-11):-

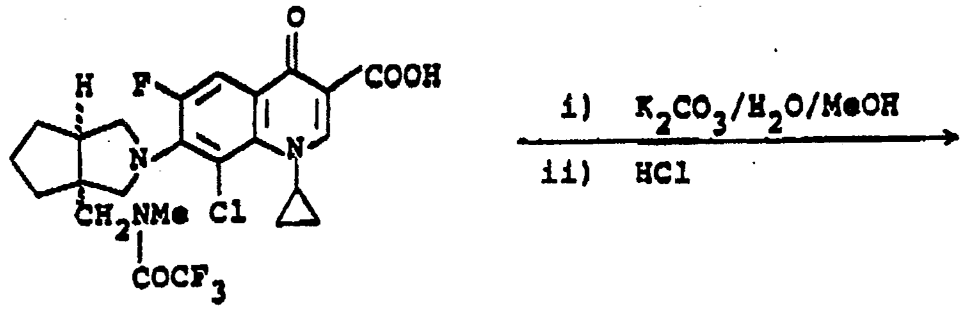


(I-1a)

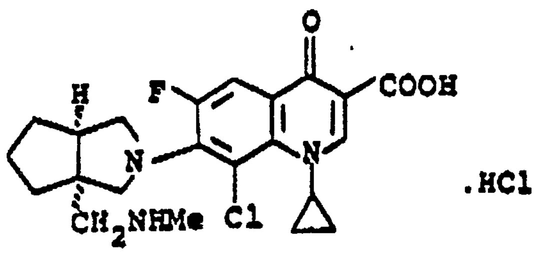

(I-11)

A solution of 7-[(1R*,5S*)-1-(N-methyl-N-trifluoroacetylaminomethyl)-3-azabicyclo[3.3.0]octan-3-yl]-1-cyclopropyl-8-chloro-6-fluoro-1,4-dihydro-4-oxo-3-quinolinecarboxylic acid (I-1a) (460 mg), potassium carbonate (480 mg), methanol (18 ml) and water (18 ml) is stirred at 50 °C for 2 hours. After removal of methanol, the reaction mixture is adjusted to pH 7 with 1N hydrochloric acid, and the precipitated crystals are collected by filtration and washed with water and ethanol. The preciptates are collected by filtration and suspended in ethanol (10 ml). To the suspension, 1N hydrochloric acid (10 ml) is added to dissolve, and the solvent is removed by distillation. The residue is dissolved in ethanol, and ethyl acetate is added thereto. The precipitates are collected by filtration and recrystallized from aqueous ethanol to give the compound (I-11) (310 mg; yield, 76 %). m.p., 233 - 235 °C (decomp.).
Elementary analysis (%) for $C_{22}H_{26}Cl_2FO_3N_3$. $1/4C_2H_5OH.2/5H_2O$:
Calcd.: C, 55.25; H, 5.83; Cl, 14.50; F, 3.88; N, 8.59.
Found: C, 54,98; H, 5.83; Cl, 14.25; F, 3.86; N, 8.96.

Examples 14 to 18

In the same manner as above, the pyridone-carboxylic acid compounds (I) as shown in Table 2 are produced.

**Table 2**

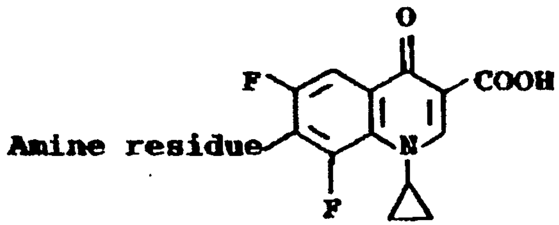

(I)

| Example No. | Compound No. | Amine residue | Z | Melting point (°C) | Molecular formula | Configuration |
|---|---|---|---|---|---|---|
| 14 | I-12 | 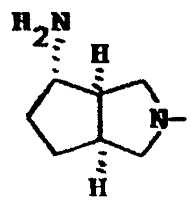 | H | 260 | $C_{20}H_{21}F_2N_3O_3$ $\cdot HCl \cdot H_2O$ | 1R*,5S*,6S* |
| 15 | I-13 | 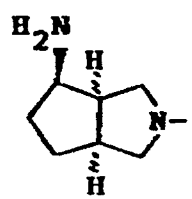 | H | 251-253 (decomp.) | $C_{20}H_{21}F_3N_2O_3$ $\cdot HCl \cdot 1/3H_2O$ | 1R*,5S*,6S* |

(Continued)

| | | | | | | |
|---|---|---|---|---|---|---|
| 16 | I-14 | 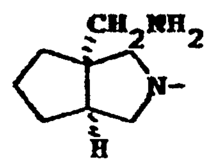 | H | 235-237 (decomp.) | $C_{21}H_{23}F_3N_3O_3$ .HCl | 1R*,5S* |
| 17 | I-15 | 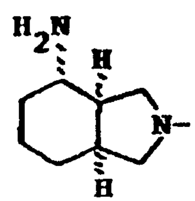 | H | 289-291 (decomp.) | $C_{21}H_{23}F_2N_3O_3$ .HCl.1/2$H_2O$ | 1R*,2R*,6R* |
| 18 | I-16 | 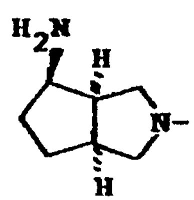 | $NH_2$ | 243 (decomp.) | $C_{20}H_{22}F_2N_4O_3$ .HCl.1/5$H_2O$ | 1R*,5S*,6R* |

Example 19

Preparation of 7-[(1R*,5S*)-1-methylaminomethyl-3-azabicyclo[3.3.0]octan-3-yl]-5-amino-1-cyclopropyl-6,8-difluoro-1,4-difluoro-4-oxo-3-quinolinecarboxylic acid hydrochoride (I-17):-

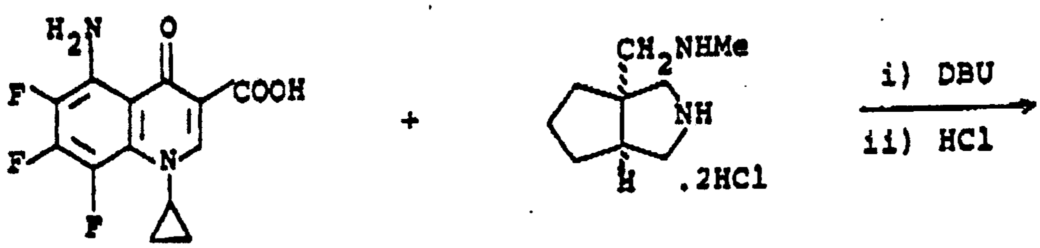


(II-4) (III-1)

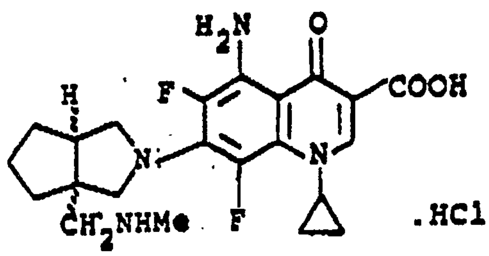

(I-17)

To a suspension of 1-amino-1-cyclopropyl-6,7,8-trifluoro-1,4-dihydro-4-oxo-3-quinolinecarboxylic acid (II-4) (1.5 g) and (1R*,5S*)-1-methylaminomethyl-3-azabicyclo[3.3.0]octane hydrochloride (III-1) (1.7 g) in acetonitrile (15 ml), DBU (3.0 ml) is added under stirring, and the resultant mixture is heated under reflux for 2 hours. After cooling, the precipitated crystals (1.7 g) are suspended in ethanol, and excess 1N hydrochloric acid is added thereto to make a solution. After removal of the solvent under reduced pressure, the residue is combined with ethanol, and the precipitated crystals are collected by filtration to give the compound (I-17) (1.7 g). m.p., 260 -262 °C (decomp.).
Elementary analysis (%) for $C_{22}H_{27}ClF_2N_4O_3.H_2O$:
Calcd.: C, 54.26; H, 6.00; Cl, 7.28; F, 7.80; N, 11.51.
Found: C, 54.38; H, 5.94; Cl, 7.05 ; F, 7.27; N, 11.45.

Example 20

Preparation of 7-[(1R*,5S*)-1-methylaminomethyl-3-azabicyclo[3.3.0]octan-3-yl]-1-cyclopropyl-6-fluoro-1,4-dihydro-4-oxo-1,8-nathylidine-3-carboxylic acid hydrochloride (I-18):-

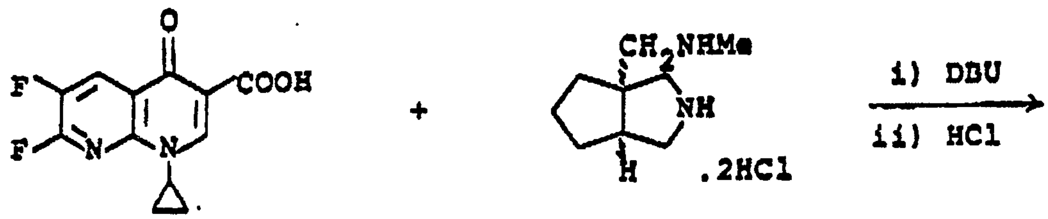


(II-5) (III-1)

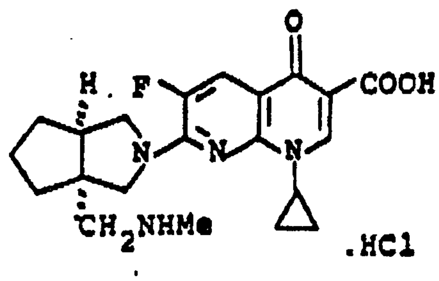

(I-18)

To a suspension of 7-chloro-1-cyclopropyl-6-fluoro-1,4-dihydro-4-oxo-1,8-naphthylidine-3-carboxylic acid (II-5) (1.0 g) and (1R*,5S*)-1-methylaminomethyl-3-azabicyclo[3.3.0]octane hydrochloride (III-1) (1.2 g) in acetonitrile (10 ml), DBU (22 g) is added under stirring, and the resultant mixture is heated under reflux for 2.5 hours. After cooling, the precipitated crystals (1.1 g) are suspended in ethanol, and excess 1N hydrochloric acid is added thereto to make a solution. After removal of the solvent under reduced pressure, the residue is combined with a mixture of ethanol and diethyl ether, and the precipitated crystals are collected by filtration to give the compound (I-18) (1.0 g). m.p., 250 - 253 ° C.
Elementary analysis (%) for $C_{21}H_{26}ClFN_4O_3.1/2H_2O$:
Calcd.: C, 56.56; H, 6.10; Cl, 7.95; F, 4.26; N, 12.57.
Found: C, 56,61; H, 5.93; Cl, 7.99; F, 3.72; N, 12.56.

Example 21

Preparation of 7-[(1R*,5S*)-1-methylaminomethyl-3-azabicyclo[3.3.0]octan-3-yl]-1-ethyl-6,8-difluoro-1,4-dihydro-4-oxo-3-quinolinecarboxylic acid hydrochloride (I-19):-

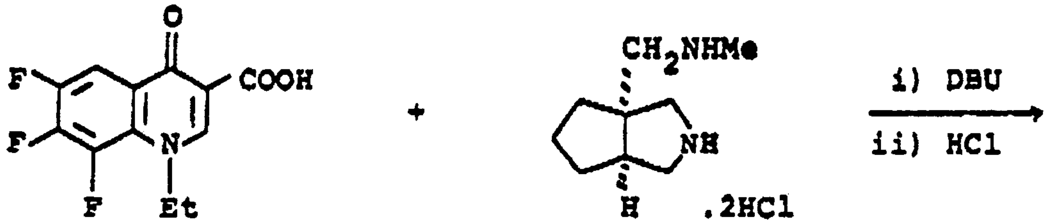


(II-6) (III-1)

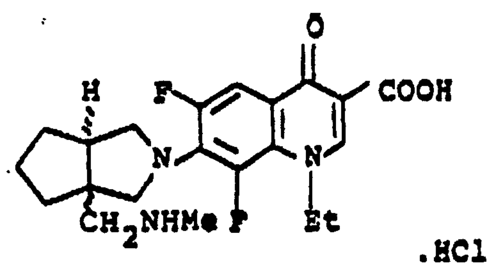

(I-19)

To a suspension of 1-ethyl-6,7,8-trifluoro-1,4-dihydro-4-oxo-3-quinolinecarboxylic acid (II-6) (1.0 g) and (1R*,5S*)-1-methylaminomethyl-3-azabicyclo[3.3.0]octane hydrochloride (III-1) (1.3 g) in acetonitrile (10 ml), DBU (6.5 ml) is added under stirring, and the resultant mixture is heated under reflux for 3 hours. After cooling, acetonitrile (10 ml) is added thereto, and the mixture is neutralized with acetic acid. The precipitated crystals (1.1 g) are suspended in methanol, and excess 10 % methanolic solution of hydrochloric acid is added thereto to make a solution. After removal of the solvent under reduced pressure, the residue is dissolved in methanol, followed by addition of ethyl acetate. The precipitated crystals (1.1 g) are recrystallized from a mixture of methanol and ethyl acetate to give the compound (I-19) (710 mg). m.p., 238 -240 ° C.

$^1$H-NMR ($D_2O$) $\delta$ (ppm): 1.51 (3H, t, J = 6.8 Hz), 1.60 - 2.00 (6H, m), 2.50 (1H, m); 2.81 (2H, s), 3.36 (1H), 3.58 (1H, d, J = 10.7 Hz), 3.71 (1H, d, J = 10.7 Hz), 3.95 (1H, m), 4.56 (2H, m), 7.82 (1H, d, J = 12.7 Hz), 8.72 (1H, s).

Example 22

Preparation of 10-[(1R*,5S*)-1-methylaminomethyl-3-azabicyclo[3.3.0]octan-3-yl]-9-fluoro-3-methyl-7-oxo-2,3-dihydro-7H-pyrido[1.2.3-de]-1,4-benzoxadine-6-carboxylic acid hydrochloride (I-20):-

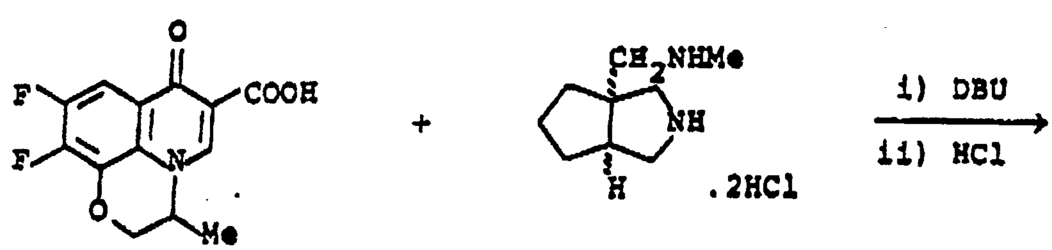


(II-7) (III-1)

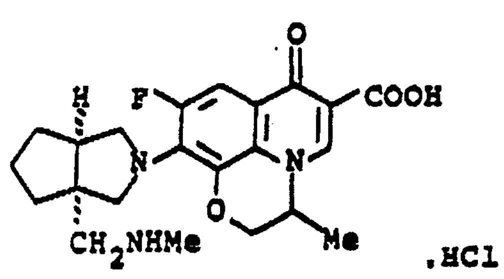

(I-20)

To a suspension of 9,10-difluoro-3-methyl-7-oxo-2,3-dihydro-7H-pyrido[1,2,3-de]-1,4-benzoxadine-6-carboxylic acid (II-7) (1.0 g) and (1R*,5S*)-1-methylaminomethyl-3-azabicyclo[3.3.0]octane hydrochloride (III-1)

(1.2 g) in acetonitrile (20 ml), DBU (2.0 ml) is added under stirring, and the resultant mixture is heated under reflux for 24 hours. After cooling, the precipitated crystals (1.2 g) are suspended in methanol, and excess 10 % methanolic solution of hydrochloric acid is added thereto to make a solution. After removal of the solvent under reduced pressure, the residue is dissolved in methanol, and ethyl acetate is added thereto. The precipitated crystals (1.1 g) are recrystallized from a mixture of methanol and ethyl acetate to give the compound (I-20) (560 mg). m.p., 222 - 223 °C (decomp.).
$^1$H-NMR ($CF_3CO_2D$) $\delta$ (ppm): 1.86 - 2.28 (9H, m), 3.07 (3H, s), 3.07 (1H, m), 3.40 (1H, m), 3.78 - 4.09 (3H, m), 4.51 - 4.92 (4H, m), 5.22 (1H, m), 8.14 (1H, d, J = 10.7 Hz), 9.39 (1H, s).

Example 23 to 28

In the same manner as above, the pyridone-carboxylic acid compounds (I) are shown in Table 3 are produced.

<u>**Table 3**</u>

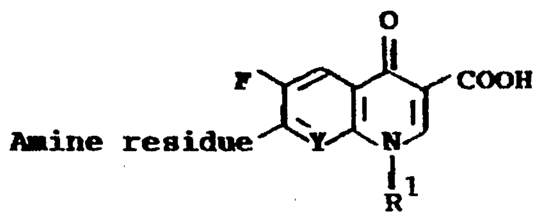

(I)

| Example No. | Compound No. | Amine residue | Y | $R^1$ | Melting point (°C) | Molecular formula | Configuration |
|---|---|---|---|---|---|---|---|
| 23 | I-21 | 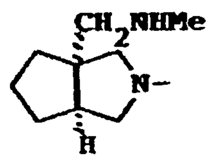 | =C(F)- | 2,4-di-F-phenyl | | | |
| 24 | I-22 | 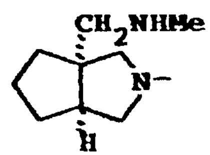 | =N- | 2,4-di-F-phenyl | | | |
| 25 | I-23 | 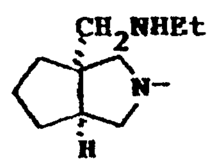 | =C(F)- | Et | 258-263 (decomp.) | $C_{22}H_{27}F_2N_3O_3$ .HCl | 1R*,5S* |

(Continued)

| | | | | | | | |
|---|---|---|---|---|---|---|---|
| 26 | I-24 | 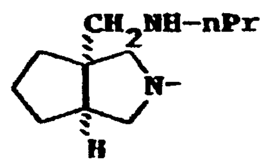 | =C(F)- | Et | 245-247 (decomp.) | $C_{23}H_{29}F_2N_3O_3$ .HCl | 1R*,5S* |
| 27 | I-25 | 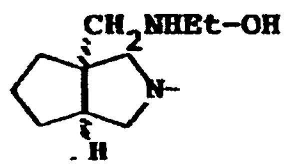 | =C(F)- | Et | 219-222 (decomp.) | $C_{22}H_{27}F_2N_3O_4$ .HCl | 1R*,5S* |
| 28 | I-26 | 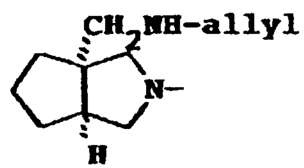 | =C(F)- | Et | 246-250 (decomp.) | $C_{23}H_{27}F_2N_3O_3$ .HCl | 1R*,5S* |

The pyridone-carboxylic acid compound (I) in a free or salt form has a strong antimicrobial activity against a wide range of microorganisms including Gram-positive bacteria, Gram-negative bacteria, mycoplasma, etc. and therefore can be used for treatment or prevention of various infectious diseases caused by them and developed in mammalian animals (e.g. cattle, pig, horse, sheep, goat, mink, mouse, rat, hamster, rabbit, gunia pig), fowls (e.g. domestic fowls, turkey, guinea fowl, quail), Amphibia (e.g. frog, newt, salamander), fish (e.g. crucian carp, goldish, killfish), etc. More specifically, the pyridone-carboxylic acid compound (I) is effective against diseases caused by mycoplasma infections, coli infections, chronic respiratory system infections, salmonellosis, fowl influenza, Pasteurella infections, etc. in poultry (e.g. chicken, turkey, guinea fowl, quail); diarrheal diseases caused by Escherichia coli, hemophilus pleuropneumonia, sepsis, dysentery, salmonellosis, arthritis, atrophic rhinitis, mycoplasma pneumonia, mesometritis, mastadenitis, erysipelas, etc. in pig; diarrheal diseases caused by Escherichia coli, sepsis, bronchopneumonia, salmonellosis, hemorrhagic septicemia, Pasteurella infections, mycoplasma infections, mastadenitis, etc. in ruminants (e.g. cattle, sheep, goat); bronchopneumonia, etc. in horse; cubitum arthritis, salmonellosis, etc. in calf; petechial bacterial hemostasis on the skin of Amphibia; nodositas, vibriosis, streptococcosis, pinnal red diseases, etc. in fish, etc.

For the practical application of the pyridone-carboxylic acid compound (I), it may be used as such or formulated into a suitable preparation form in combination with a suitable carrier(s) or adjuvant(s) and, if necessary, also with an auxiliary agent(s) such as a decomposing agent, a lubricant, a stablizer, a flavouring agent, a pigment, a preservative or an aromatic agent. Examples of the preparation form are dispersants, granules, solutions, emulsions, suspensions, premixes, capsules, emulsifiable concentrates, tablets and so forth. As the carrier(s) or adjuvant(s), there are usable water, gum arabic, lactose, sucrose, talc, colloidal silica, soybean oil, starch, yeast, wheat, defatted soybean, corn, wheat bran, commercial bait, etc. Further, the pyridone-carboxylic acid compound (I) may be mixed with one or more other veterinary medicine(s) to form an associated composition.

The mode of administration may be appropriately decided depending upon the kind of the active component, the state of the disease, the sort of the animal or fish, etc. When, for instance, the pyridone-carboxylic acid compound (I) is used for treatment or prevention of mycoplasma infections, the composition containing the same may be applied parenterally or non-parenterally, particularly per os or by inection (e.g. intramuscular injection, intravenous injection, subcutaneous injection). The oral dose of the active component may be from about 0.1 to 100 mg/kg/day. On the application, the composition is normally diluted with water or mixed with a bait so as to have an active concentration of about 5 to 1,000 ppm. In case of injection, the active component may be used in an amount of from about 0.1 to 10 mg/kg/day.

Practical embodiments of the veterinary medicinal composition according to the invention are shown below, in which part(s) and % are by weight unless otherwise indicated.

Preparation Example 1

Compound No. (I-1) (500, 400, 200, 100 or 50 mg) is dissolved in water (1000 ml) to make a drinking solution each containing 0.05, 0.04, 0.02, 0.01 or 0.005 % (w/v) of the active component.

Preparation Example 2

Compound No. (I-2) (500, 400, 200, 100 or 50 mg) is mixed with a commercial bait (1,000 g) to make a bait each containing 500, 400, 200, 100 and 50 ppm of the active component. The commercial bait used in this Example contains the following ingredients: corn, 41.00 %; milo (wheat extract), 25.00 %; soybean cake, 19.10 %; fish powders, 8.00 %, oil, 4.00 %; sodium carbonate, 1.40 %; calcium phosphate, 0.85 %; a mixture of vitamin-inorganic salts (Vitamin A, Vitamin $D_3$, Vitamin E, Vitamin $B_1$, Vitamin $B_2$, Vitamin $B_6$, Vitamin $B_{12}$, calcium D-panththenate, nicotinamide, Vitamin $K_4$, choline chloride, magnesium sulfate, iron sulfate, copper sulfate, zinc sulfate, cobalt sulfate, potassium iodide), 0.26 %; methionine, 0.10 %, sodium chloride, 0.29 %.

Preparation Example 3

Compound No. (I-5) (500, 400, 200, 100 or 50 mg) is dissolved in an aqueous medium (25 ml)

comprising 3 % (w/v) gum arabic, 5 % gum arabic, 3 % gum arabic + 20 mg/ml sodium citrate, 20 % ethanol, 20 mg/ml sodium citrate, sodium carboxymethyl cellulose (10 g) + Tween 80 (8 g) + benzyl alcohol (18 g) + sodium chloride (18 g) per 2 liters of distilled water, 20 % dimethylsulfoxide (DMSO) or distilled water to make an aqueous solution for oral administration each containing 20, 10 and 5 mg/ml of the active component.

The antimicrobial activity of the pyridone-carboxylic acid compound (I) is illustratively shown in the following Test Examples.

Test Example 1

Sensitivity test in vitro:-

Survey of the sensitivity of the compound (I) to various strains of microorganisms was performed by a liquid dilution method of an agar dilution method, in which the bacteria used were of animal origin and included the following strains (15 strains out of 11 bacteria): Chicken-origin strain: Mycoplasma gallisepticum (MG), Mycoplasma synoviae (MS), Hemophilus paragallinarum (H.pg) and Escherichia coli (E.coli); Pig-origin strain: Mycoplasma hyopneumoniae (Mh), Hemophilus pleuropneumonia (H.pp) and Bordetella bronchiseptica (B.br); Fish-origin strain: Pasteurella piscicida (P.pis) and Streptococcus sp (Str.sp); others: Escherichia coli (E. coli), Salmonella typhimurium (S.typhi) and Staphylococcus aureus (S.au).

Of these bacteria, the strains belonging to MG, MS and Mh were cultured at 37°C for 7 days by a liquid dilution method, of which the culture medium comprised: 12 % horse-serum added "PPLO" (agar manufactured by Difco) medium for MG; 12 % pig-serum, 0.01 % βNAD (nicotinamide adenine dinucleotide) added Frey medium for MS; 0.5 % lactoalbumin added Hank's solution, 10 % horse-serum, 0.5 % yeast extract (25 %) for Mh, and the agar dilution method was applied to the strains belonging to H.pg, H.pp, S.typhi, E.coli, S.au and B.br. Specifically, the H.pg strain was cultured in a 5 % chicken-serum added chicken bouillon medium and the H.pp strain was cultured in a heart infusion agar added with 10 % sheep defibered blood and 0.05 % βNAD. In case of the H.pg and P.pp strains, observation was made after cultivation at 37°C for 20 hours under 10 % $CO_2$. Mueller Hinton agar (Difco) was used for the strains belonging to S.typhi, E.coli, S.au and B.br, and observation was made after cultivation at 37°C for 20 to 24 hours. P.pis and Str.sp, both being of fish-origin, were respectively cultured at 25°C for 40 to 48 hours in a 1.5 % NaCl added heart infusion agar and in a sensitive disk medium.

The results of the above survey, i.e. sensitivity of the compound (I) to the animal-origin bacteria are shown in Table 4. For comparison, Enrofuroxasin® (ERFX; Bayeruro Co.) was used as the control drug, and Tylosin® (TSP; Tylosin Premix-20; 2 % titer), chlorotetracycline hydrochloride (CTC; CT Cycline-100, 10 % titer, Shionogi & Co.) and doxycycline (DOXY) were employed as the commercial antimicrobial agents.

Table 4

In vitro Sensitivity Test (μg/ml)

| Test compound | MG | | MS | | Mh | E.coli | | H.pg | | S.typhi | S.au | H.pp | B.br | P.pis | Str.sp |
|---|---|---|---|---|---|---|---|---|---|---|---|---|---|---|---|
| | S6 | T-7-T*1) | 1853 | F10-2as | ST-11 | JC-2 | E-17*2) | 221 | S-1 | 13311 | 209P | M-1 | ABU-1 | No. 2 | SNO6119 |
| I-1 | 0.0125 | 0.025 | - | - | - | 0.2 | 0.2 | 0.78 | 0.78 | 0.2 | 0.025 | 0.39 | 12.5 | 0.78 | 0.78 |
| I-2 | 0.025 | 0.05 | - | - | 0.1 | 0.2 | 0.2 | 3.13 | 3.13 | 0.2 | 0.2 | 0.39 | 12.5 | 6.25 | 3.13 |
| I-3 | 0.025 | 0.05 | - | - | 0.05 | 0.1 | 0.1 | 1.56 | 1.56 | 0.1 | 0.2 | 0.2 | 6.25 | 1.56 | 0.78 |
| I-4 | 0.05 | - | - | - | ≦0.025 | 0.2 | - | - | - | 0.39 | 0.05 | 0.2 | - | 6.25 | 0.39 |
| I-5 | 0.00625 | 0.00625 | 0.78 | 0.2 | <0.0025 | 0.1 | 0.1 | 0.2 | 0.2 | 0.2 | 0.1 | 0.2 | 6.25 | 1.56 | 0.78 |
| I-6 | 0.00625 | 0.00625 | 0.2 | 0.2 | <0.025 | 0.1 | 0.2 | >1.56 | >1.56 | 0.2 | ≦0.0125 | 0.2 | 3.13 | 0.78 | 0.39 |
| I-7 | 0.05 | 0.2 | - | - | 0.1 | 0.39 | 0.39 | >3.13 | >3.13 | 0.2 | 0.78 | 0.78 | 6.25 | 6.25 | 3.13 |
| I-8 | <0.00313 | 0.00625 | 0.1 | 0.1 | <0.025 | 0.05 | 0.05 | 0.2 | 0.2 | 0.2 | <0.0125 | 0.05 | 1.56 | 0.78 | 0.78 |
| I-10 | 0.00625 | - | - | - | 0.05 | 0.78 | 0.39 | 0.78 | 0.39 | 0.78 | 0.025-0.05 | 0.78 | 12.5 | 1.56 | 0.78 |
| I-12 | 0.00313 | - | - | - | <0.00625 | 0.1 | - | - | - | 0.1 | 0.1 | 0.2 | 0.39 | 0.39 | 0.78 |
| I-13 | 0.00313 | - | ≦0.025 | 0.05 | <0.00625 | 0.025 | - | - | - | 0.025 | <0.0125 | 0.39 | 0.39 | 0.2 | 0.2 |
| I-14 | <0.00313 | 0.00313 | 0.2 | 0.1 | <0.025 | 0.1 | 0.05 | 0.1 | 0.1 | 0.1 | 0.025 | 0.1 | 1.56 | 0.39 | 0.2 |
| I-15 | <0.00313 | 0.00313 | 0.1 | 0.1 | <0.025 | 0.05 | 0.025 | 0.2 | 0.2 | 0.05 | 0.025 | 0.1 | 0.39 | 0.39 | 0.39 |
| I-16 | <0.0313 | 0.00313 | 0.39 | 0.2 | <0.025 | 0.05 | 0.025 | 0.2 | 0.2 | 0.05 | 0.025 | 0.2 | 1.56 | 0.39 | 0.39 |
| I-19 | 0.00625 | - | - | - | 0.39 | 0.39 | - | - | - | 0.78 | 0.05 | 0.78 | - | 1.56 | 1.56 |
| I-23 | 0.0125 | - | - | - | 0.78 | 0.78 | - | - | - | 1.56 | 0.1 | 0.78 | - | 0.78 | 0.39 |
| EAPX | 0.1 | 0.1 | 0.1 | 3.13 | 0.05 | 0.1 | 0.1 | 0.1 | 0.2 | 0.1 | 0.1 | 0.2 | 0.1 | - | - |
| TS | 0.05 | 6.25 | 0.1 | 0.1 | 0.1 | 25.0 | - | - | - | 0.39 | 0.39 | 25.0 | - | - | - |
| CTC | 12.5 | 6.25 | 0.05 | 0.39 | 1.56 | 0.39 | - | - | - | 0.1 | 0.1 | - | - | - | - |
| DOXY | 0.2 | 0.2 | 0.1 | 0.39 | - | 0.39 | - | - | - | 0.1 | 0.1 | 0.39 | - | - | - |

Note: *1) TS-resistant strain isolated from the field
*2) chicken-origin strain isolated from the field

It is understood from the results of Table 4 that the compounds (I) according to the invention are more sentitive than the commercial antimicrobial agents. Further, it is particularly notable that the compounds (I) are highly sensitive to the microorganisms belonging to mycoplasma, i.e. MG and Mn, than the control drug ERFX.

Test Example 2

In vivo survey on the experimentally infected diseases caused by Mg, Hpg and H.pp:-

Chickens and pigs were experimentally infected respectively with MG and H.pg, and H.pp, and the controlling effect of the compound (I) on the infectirous diseases was observed. In these experiments, the compound (I) was applied to the designated animals by (1) oral administration, (2) incorporation into baits and (3) intramuscular injection, the details of which are explained below.

(1) Specific pathogen free (SPF) chickens raised at Aburahi Laboratory, Shiga-ken, Japan, were infected with the strains of MG. In case of MG, some 9 to 10 days chickens (each group being 4 or 5 fowls with no sexual discrimination) were infected with a TS-resistant strain of MG, which was isolated by the in vitro cultivation at 37 °C for 1 to 2 days and diluted with a fresh medium, at the right air sac in an amount of about $10^2$ to $10^3$ colony forming unit (CFU), followed by immediate administration of the designated compound. The chickens were raised in a cage kept at 25 ± 2 °C and dissected on the 5th day.

The antimicrobial activity of the test compounds through oral administration is shown in Tables 5 (10 days SPF chicken; 1.2 x $10^3$ CFU/fowl), 6 (10 days SPF chicken; 3.0 x $10^3$ CFU/fowl), 7 (9 days SPF chicken; 1.7 x $10^3$ CFU/fowl), 8 (9 days SPF chicken; 2.2 x $10^3$ CFU/fowl). and 9 (9 days SPF chicken; 3.5 x $10^3$ CFU/fowl). The infectious status was evaluated on the following criteria: -, no infection; +, slight infection; + +, considerable infection; + + +, severe infection.

Table 5

| Antimicrobial activity on experimental MG infection to chicken through oral administration (10-days chicken infected with $1.2 \times 10^3$ CFU/fowl) | | | | | | | | | | | |
|---|---|---|---|---|---|---|---|---|---|---|---|
| Test compound | Dose (mg/kg) | Number of fowl tested | Infectious index | | | | | | | | Number of fowl having air sack lesions |
| | | | Right air sack | | | | Left air sack | | | | |
| | | | - | + | + + | + + + | - | + | + + | + + + | |
| I-1 | 3.13 | 5 | 0 | 1 | 3 | 1 | 4 | 1 | 0 | 0 | 5 |
| | 6.25 | 5 | 1 | 3 | 1 | 0 | 5 | 0 | 0 | 0 | 4 |
| | 12.5 | 5 | 4 | 0 | 1 | 0 | 5 | 0 | 0 | 0 | 1 |
| | 25.0 | 5 | 5 | 0 | 0 | 0 | 5 | 0 | 0 | 0 | 0 |
| I-5 | 1.56 | 4 | 2 | 0 | 1 | 1 | 4 | 0 | 0 | 0 | 2 |
| | 3.13 | 5 | 5 | 0 | 0 | 0 | 5 | 0 | 0 | 0 | 0 |
| | 6.25 | 5 | 5 | 0 | 0 | 0 | 5 | 0 | 0 | 0 | 0 |
| | 25.0 | 5 | 5 | 0 | 0 | 0 | 5 | 0 | 0 | 0 | 0 |
| I-6 | 3.13 | 5 | 5 | 0 | 0 | 0 | 5 | 0 | 0 | 0 | 0 |
| | 6.25 | 5 | 0 | 1 | 4 | 0 | 4 | 1 | 0 | 0 | 5 |
| | 12.5 | 5 | 0 | 0 | 4 | 1 | 5 | 0 | 0 | 0 | 5 |
| I-8 | 1.56 | 5 | 0 | 3 | 2 | 0 | 5 | 0 | 0 | 0 | 5 |
| | 3.13 | 5 | 4 | 1 | 0 | 0 | 5 | 0 | 0 | 0 | 1 |
| | 6.25 | 5 | 5 | 0 | 0 | 0 | 5 | 0 | 0 | 0 | 0 |
| ERFX | 6.25 | 5 | 3 | 1 | 1 | 0 | 5 | 0 | 0 | 0 | 2 |
| | 12.5 | 5 | 5 | 0 | 0 | 0 | 5 | 0 | 0 | 0 | 0 |
| | 25.0 | 5 | 5 | 0 | 0 | 0 | 5 | 0 | 0 | 0 | 0 |
| DOXY | 100 | 5 | 4 | 0 | 1 | 0 | 5 | 0 | 0 | 0 | 1 |
| | 200 | 5 | 4 | 0 | 1 | 0 | 4 | 1 | 0 | 0 | 1 |
| No treatment with drug | - | 5 | 0 | 1 | 1 | 3 | 4 | 1 | 0 | 0 | 5 |
| Non-infected | - | 5 | 5 | 0 | 0 | 0 | 5 | 0 | 0 | 0 | 0 |

Table 6

| Antimicrobial activity on experimental MG infection to chicken through oral administration (10-days chicken infected with 3.0 x $10^3$ CFU-fowl) | | | | | | | | | | | |
|---|---|---|---|---|---|---|---|---|---|---|---|
| Test compound | Dose (mg/kg) | Number of fowl tested | Infectious index | | | | | | | | Number of fowl having air sack lesions |
| | | | Right air sack | | | | Left air sack | | | | |
| | | | - | + | + + | + + + | - | + | + + | + + + | |
| I-2 | 3.13 | 5 | 1 | 0 | 4 | 0 | 5 | 0 | 0 | 0 | 4 |
| | 6.25 | 5 | 3 | 1 | 1 | 0 | 4 | 1 | 0 | 0 | 4 |
| | 12.5 | 5 | 4 | 0 | 1 | 0 | 5 | 0 | 0 | 0 | 1 |
| I-3 | 3.13 | 5 | 2 | 0 | 3 | 0 | 4 | 0 | 1 | 0 | 3 |
| | 6.25 | 5 | 0 | 2 | 3 | 0 | 5 | 0 | 0 | 0 | 5 |
| | 12.5 | 5 | 2 | 2 | 1 | 0 | 4 | 1 | 0 | 0 | 3 |
| I-7 | 3.13 | 5 | 1 | 0 | 3 | 1 | 5 | 0 | 0 | 0 | 4 |
| | 6.25 | 5 | 0 | 1 | 3 | 1 | 5 | 1 | 0 | 0 | 5 |
| | 12.5 | 5 | 0 | 2 | 1 | 2 | 5 | 0 | 0 | 0 | 5 |
| ERFX | 6.25 | 5 | 3 | 1 | 1 | 0 | 5 | 0 | 0 | 0 | 2 |
| | 12.5 | 5 | 5 | 0 | 0 | 0 | 5 | 0 | 0 | 0 | 0 |
| | 25.0 | 5 | 5 | 0 | 0 | 0 | 5 | 0 | 0 | 0 | 0 |
| DOXY | 100 | 5 | 4 | 0 | 1 | 0 | 5 | 0 | 0 | 0 | 1 |
| | 200 | 5 | 4 | 0 | 1 | 0 | 4 | 1 | 0 | 0 | 5 |
| No treatment with drug | - | 5 | 0 | 1 | 1 | 3 | 4 | 1 | 0 | 0 | 5 |
| Non-infected | - | 5 | 5 | 0 | 0 | 0 | 5 | 0 | 0 | 0 | 0 |

Table 7

| Antimicrobial activity on experimental MG infection to chicken through oral administration (9-days chicken infected with 1.7 x $10^3$ CFU/fowl) | | | | | | | | | | | |
|---|---|---|---|---|---|---|---|---|---|---|---|
| Test compound | Dose (mg/kg) | Number of fowl tested | Infectious index | | | | | | | | Number of fowl having air sack lesions |
| | | | Right air sack | | | | Left air sack | | | | |
| | | | - | + | + + | + + + | - | + | + + | + + + | |
| I-4 | 6.25 | 4 | 2 | 1 | 1 | 0 | 3 | 1 | 0 | 0 | 2 |
| | 12.5 | 5 | 5 | 0 | 0 | 0 | 5 | 0 | 0 | 0 | 0 |
| | 25.0 | 5 | 5 | 0 | 0 | 0 | 5 | 0 | 0 | 0 | 0 |
| I-15 | 6.25 | 4 | 4 | 0 | 0 | 0 | 4 | 0 | 0 | 0 | 0 |
| | 12.5 | 5 | 5 | 0 | 0 | 0 | 5 | 0 | 0 | 0 | 0 |
| | 25.0 | 5 | 5 | 0 | 0 | 0 | 5 | 0 | 0 | 0 | 0 |
| I-16 | 6.25 | 4 | 1 | 0 | 3 | 0 | 4 | 0 | 0 | 0 | 3 |
| | 12.5 | 5 | 5 | 0 | 0 | 0 | 5 | 0 | 0 | 0 | 0 |
| | 25.0 | 5 | 5 | 0 | 0 | 0 | 5 | 0 | 0 | 0 | 0 |
| ERFX | 6.25 | 4 | 3 | 0 | 1 | 0 | 4 | 0 | 0 | 0 | 1 |
| | 12.5 | 5 | 5 | 0 | 0 | 0 | 5 | 0 | 0 | 0 | 0 |
| | 25.0 | 5 | 5 | 0 | 0 | 0 | 5 | 0 | 0 | 0 | 0 |
| DOXY | 100 | 5 | 4 | 0 | 1 | 0 | 5 | 0 | 0 | 0 | 1 |
| | 200 | 5 | 4 | 0 | 1 | 0 | 4 | 1 | 0 | 0 | 1 |
| No treatment with drug | - | 5 | 0 | 1 | 4 | 0 | 3 | 1 | 1 | 0 | 5 |
| Non-infected | - | 5 | 5 | 0 | 0 | 0 | 5 | 0 | 0 | 0 | 0 |

Table 8

| Antimicrobial activity on experimental MG infection to chicken through oral administration (9-days chicken infected with 2.2 x $10^3$ CFU/fowl) | | | | | | | | | | | |
|---|---|---|---|---|---|---|---|---|---|---|---|
| Test compound | Dose (mg/kg) | Number of fowl tested | Infectious index | | | | | | | | Number of fowl having air sack lesions |
| | | | Right air sack | | | | Left air sack | | | | |
| | | | - | + | + + | + + + | - | + | + + | + + + | |
| I-10 | 1.56 | 4 | 1 | 2 | 0 | 1 | 4 | 0 | 0 | 0 | 3 |
| | 6.25 | 5 | 1 | 2 | 2 | 0 | 4 | 1 | 0 | 0 | 4 |
| | 12.5 | 5 | 5 | 0 | 0 | 0 | 5 | 0 | 0 | 0 | 0 |
| | 25.0 | 5 | 5 | 0 | 0 | 0 | 5 | 0 | 0 | 0 | 0 |
| ERFX | 3.13 | 5 | 3 | 1 | 0 | 1 | 5 | 0 | 0 | 0 | 2 |
| | 6.25 | 5 | 5 | 0 | 0 | 0 | 5 | 0 | 0 | 0 | 0 |
| | 12.5 | 5 | 5 | 0 | 0 | 0 | 5 | 0 | 0 | 0 | 0 |
| DOXY | 100 | 5 | 4 | 0 | 1 | 0 | 5 | 0 | 0 | 0 | 1 |
| | 200 | 5 | 4 | 0 | 1 | 0 | 4 | 1 | 0 | 0 | 1 |
| No treatment with drug | - | 5 | 0 | 2 | 3 | 0 | 5 | 0 | 0 | 0 | 5 |
| Non-infected | - | 5 | 5 | 0 | 0 | 0 | 5 | 0 | 0 | 0 | 0 |

Table 9

| Antimicrobial activity on experimental MG infection to chicken through oral administration (9-days chicken infected with 3.5 x $10^3$ CFU/fowl) | | | |
|---|---|---|---|
| Test compound | Dose (mg/kg) | Number of fowl tested | Number of infected fowl/Number of fowl tested |
| I-16 | 1.56 | 5 | 5/5 |
| | 3.13 | 5 | 2/5 |
| | 6.25 | 5 | 2/5 |
| I-19 | 1.56 | 5 | 3/5 |
| | 3.13 | 5 | 3/5 |
| | 6.25 | 5 | 1/5 |
| I-23 | 1.56 | 5 | 5/5 |
| | 3.13 | 5 | 5/5 |
| | 6.25 | 5 | 0/5 |
| ERFX | 1.56 | 5 | 2/5 |
| | 3.13 | 5 | 0/5 |
| | 6.25 | 5 | 0/5 |
| No treatment with drug | - | 5 | 5/5 |
| Non-infected | - | 5 | 0/5 |

It is understood from Tables 5 through 9 that the antimicrobial activity of the compounds (I) according to the invention is better than that of ERFX. Of these, Compounds Nos. (I-5), (I-8) and (I-12) are two times more effective than ERFX and produced complete inihibition of the lesions in air sac at a concentration of about 3.13 mg/kg (12.5 mg/kg for ERFX). Compounds Nos. (I-1), (I-11), (I-13) and (I-14) showed a nearly equal potency to ERFX. All of the compounds (I) exhibited a superior antimicrobial activity to DOXY, which is a commercially available antimicrobial agent.

(2) Preparation of the MG infected fowls was performed in the same manner as in (1) above. In case of H.pg, a renewed dilute medium of the H.pg strain (i.e. 221), which causes infectious coryza, was inoculated to the nasopharyngeal of some 28 day old chickens (each group being 7 fowls) at 4 x $10^6$ CFU, and the chickens were raised with the bait containing the test compounds for 3 days. Dissection of the chickens was effected ten days after the inoculation. The chickens infected with MG and H.pg were raised in a cage kept at 25 ± 2 $^\circ$C until dissection. Evaluation of the antimicrobial activity was made by observation of infectious status of the air sac in connection with MG in the same manner as in (1) and an amount of pituita and a facial swelling degree with regard to H.pg during the administration.

The antimicrobial activity of the test compounds through the bait is shown in Tables 10 (bacteria: TS-resistant MG; 9 days SPF chicken; 1.8 x $10^3$ CFU/fowl; dissection, 6 days after inoculation), 11 (bacteria: TS-resistant MG; 9 days chicken; 6.6 x $10^3$ CFU/fowl; dissection, 6 days after inoculation) and 12 (28 days SPF chicken; 4 x $10^8$ CFU/fowl; dissection, 10 days after inoculation).

Table 10

| Antimicrobial activity on experimental MG infection to chicken through bait (9-days chicken infected with $1.8 \times 10^3$ CFU/fowl) | | | | | | | | | | | |
|---|---|---|---|---|---|---|---|---|---|---|---|
| Test compound | Dose (ppm)/Day | Number of fowl tested | Infectious index | | | | | | | | Number of fowl having air sack lesions |
| | | | Right air sack | | | | Left air sack | | | | |
| | | | - | + | + + | + + + | - | + | + + | + + + | |
| I-5 | 7.8 | 5 | 3 | 2 | 0 | 0 | 5 | 0 | 0 | 0 | 2 |
| | 15.6 | 5 | 5 | 0 | 0 | 0 | 5 | 0 | 0 | 0 | 0 |
| | 31.3 | 5 | 5 | 0 | 0 | 0 | 5 | 0 | 0 | 0 | 0 |
| I-14 | 15.6 | 5 | 1 | 0 | 4 | 0 | 5 | 0 | 0 | 0 | 4 |
| | 31.3 | 5 | 5 | 0 | 0 | 0 | 5 | 0 | 0 | 0 | 0 |
| | 62.5 | 5 | 5 | 0 | 0 | 0 | 5 | 0 | 0 | 0 | 0 |
| I-15 | 15.6 | 5 | 1 | 3 | 1 | 0 | 5 | 0 | 0 | 0 | 4 |
| | 31.3 | 5 | 4 | 1 | 0 | 0 | 5 | 0 | 0 | 0 | 1 |
| | 62.5 | 5 | 5 | 0 | 0 | 0 | 5 | 0 | 0 | 0 | 0 |
| I-16 | 15.6 | 5 | 2 | 1 | 2 | 0 | 5 | 0 | 0 | 0 | 3 |
| | 31.3 | 5 | 2 | 1 | 2 | 0 | 5 | 0 | 0 | 0 | 3 |
| | 62.5 | 5 | 5 | 0 | 0 | 0 | 5 | 0 | 0 | 0 | 0 |
| ERFX | 15.6 | 5 | 4 | 0 | 1 | 0 | 5 | 0 | 0 | 0 | 1 |
| | 31.3 | 5 | 5 | 0 | 0 | 0 | 5 | 0 | 0 | 0 | 0 |
| | 62.5 | 5 | 5 | 0 | 0 | 0 | 5 | 0 | 0 | 0 | 0 |
| TSP | 1100 | 5 | 0 | 3 | 2 | 0 | 4 | 1 | 0 | 0 | 5 |
| CTC | 880 | 5 | 1 | 3 | 1 | 0 | 5 | 0 | 0 | 0 | 4 |
| No treatment with drug | - | 5 | 0 | 1 | 4 | 0 | 3 | 1 | 1 | 0 | 5 |
| Non-infected | - | 5 | 5 | 5 | 0 | 0 | 0 | 5 | 0 | 0 | 0 |

It is understood from Table 10 that the antimicrobial activity of the compounds (I) according to the invention is superior to those of ERFX and the commercially available TSP and CTC. Of these, Compound No. (I-5) (15.6 ppm) was two times more effective than EFFX, and Compounds Nos. (I-14) and (I-15) (31.3 ppm) showed the same level as ERFX. TPC and CTC as commercially available antimicrobial agents could not inhibit lesions in air sacs at such a high concentration as 1100 ppm and 880 ppm, respectively.

Table 11

| Antimicrobial activity on experimental MG infection to chicken through bait (9-days chicken infected with 6.6 x $10^3$ CFU/fowl; dissection, 6th day after inoculation) | | | |
|---|---|---|---|
| Test compound | Dose (ppm)/Day | Number of fowl tested | Number of infected fowl/Number of fowl tested |
| I-16 | 3.13 | 7 | 2/7 |
| | 62.5 | 7 | 0/7 |
| | 125 | 7 | 0/7 |
| I-19 | 31.3 | 7 | 1/7 |
| | 62.5 | 7 | 0/7 |
| | 125 | 7 | 0/7 |
| I-23 | 31.3 | 7 | 5/7 |
| | 62.5 | 7 | 0/7 |
| | 125 | 7 | 0/7 |
| ERFX | 31.3 | 7 | 1/7 |
| | 62.5 | 7 | 0/7 |
| | 125 | 7 | 0/7 |
| No treatment with drug | - | 7 | 7/7 |
| Non-infected | - | 5 | 0/5 |

Table 12

| Antimicrobial activity on experimental H.pg tion tion to chicken through bait (28-days chicken infected with 4 x $10^8$ CFU/fowl; dissection, 10th day after inoculation) | | |
|---|---|---|
| Test compound | Effective concentration to prevent pituita (ppm/day) | Number of infected fowl/Number of fowl tested |
| I-5 | 1.56 | 0/7 |
| ERFX | 7.8 | 0/7 |
| No treatment with drug | - | 7/7 |
| Non-infected | - | 0/7 |

(3) Preparation of the MG infected fowls was performed in the same manner as in (1) above. Infection of H.pp (otherwise called Actinobacillus pleuropneumoniae) to cause pleuropneumonia in pig was performed by inoculating 5 ml of a fresh cultured strain (i.e. M-1) of H.pp to each nasopharyngeal of some 45 days pigs, each weighing 4 to 9 kg (4 to 5 pigs/group), followed by injection of the test compounds to each thigh muscle at a designated volume one time. Pigs were dissected 6 days after the inoculation. The evaluation of the antimicrobial activity was made by observation of the pneumonia symptomes in air sacs, number of dead body and degree of nodosity in air sacks on dissection.

The antimicrobial activity of the test compounds through injection is shown in Tables 13 (bacteria: TS-resistant MG; 10 days SPF chicken; 3.1 x $10^3$ CFU/fowl; dissection, 5 days after inoculation) and 14 (bacteria: H.pp; pig of about 4 to 9 kg weight; $10^8$ CFU/ml, dissection, 6 days after inoculation).

Table 13

| Antimicrobial activity on experimental MG infection to chicken through injection (10-days chicken infected with 3.1 x $10^3$ CFU/fowl) | | | | | | | | | | | |
|---|---|---|---|---|---|---|---|---|---|---|---|
| Test compound | Dose (mg/kg) | Number of fowl tested | Infectious index | | | | | | | | Number of fowl having air sack lesions |
| | | | Right air sack | | | | Left air sack | | | | |
| | | | - | + | + + | + + + | - | + | + + | + + + | |
| I-14 | 1.56 | 5 | 5 | 0 | 0 | 0 | 5 | 0 | 0 | 0 | 0 |
| | 3.13 | 5 | 5 | 0 | 0 | 0 | 5 | 0 | 0 | 0 | 0 |
| I-15 | 1.56 | 5 | 5 | 0 | 0 | 0 | 5 | 0 | 0 | 0 | 0 |
| | 3.13 | 5 | 5 | 0 | 0 | 0 | 5 | 0 | 0 | 0 | 0 |
| I-6 | 1.56 | 5 | 5 | 0 | 0 | 0 | 5 | 0 | 0 | 0 | 0 |
| | 3.13 | 5 | 0 | 0 | 0 | 0 | 5 | 0 | 0 | 0 | 0 |
| ERFX | 1.56 | 5 | 2 | 1 | 2 | 0 | 5 | 0 | 0 | 0 | 3 |
| | 3.13 | 5 | 3 | 1 | 1 | 0 | 5 | 0 | 0 | 0 | 2 |
| No treatment with drug | - | 5 | 0 | 2 | 3 | 0 | 5 | 0 | 0 | 0 | 5 |
| Non-infected | - | 5 | 5 | 0 | 0 | 0 | 5 | 0 | 0 | 0 | 0 |

Table 14

| Antimicrobial activity on experimental H.pp infection to pig through injection | | | |
|---|---|---|---|
| Test compound | Dose (mg/kg) | Number of pig died or developed dyspnea/Number of pig tested | Number of pig having nodi/Number of pig tested |
| I-5 | 10 | 0/4 | 0/4 |
| I-16 | 5 | | 0/4 |
| I-23 | 5 | | 2/4 |
| ERFX | 5 | 0/4 | 0/4 |
| | 10 | 0/4 | 0/4 |
| No treatment with drug | - | - | 4/5 |
| Non-infected | - | - | 0/5 |

(4) Toxicity:-

The compounds (I) according to the invention show a higher antimicrobial activity than the control or commercially available antimicrobial agents with a smaller dosage. As shown in Table 15, the acute-toxicity ($LD_{50}$) of the compounds (I) is extremely low, so that those may be used safely.

Table 15

| Toxicity | | | | | |
|---|---|---|---|---|---|
| Compound No. | $LD_{50}$ (mg/kg) | | | | |
| | Mouse ($B6C3F_1$, male) | | Mouse (DS, male) | | Chicken ( -days) |
| | p.o. | i.p. | p.o. | i.p. | p.o. |
| I-5 | 2000 | 200 | 2000 | 200 | 500 |
| Note: p.o., per oral; i.p., intraperitoneal | | | | | |

## Claims

1. A veterinary composition for the treatment or prophylaxis of infectious diseases caused by infectious microorganisms which comprises at least one pyridone-carboxylic acid compound of the formula:

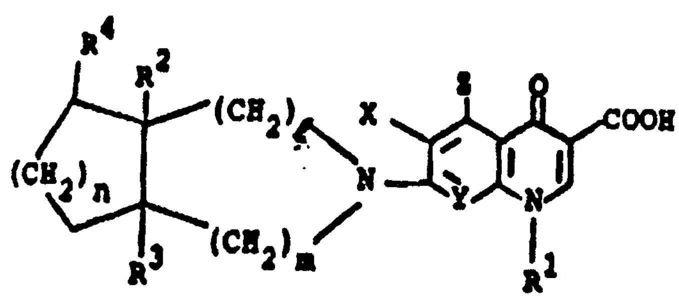

wherein
$R^1$ is lower alkyl, halo(lower)alkyl, cyclo(lower) alkyl or substituted or unsustituted phenyl;
$R^2$ and $R^3$ are each hydrogen, hydroxyl, lower alkoxy, halogen or a group of the formula: $-CH_2N(R^5)R^6$ (in which $R^5$ and $R^6$ are each hydrogen, lower alkyl, lower alkenyl or hydroxy(lower)alkyl);
$R^4$ is hydrogen, oxo, hydroxyl, lower alkoxy, halogen or a group of the formula: $-CH_2N(R^7)R^8$ (in which $R^7$ and $R^8$ are each hydrogen or lower alkyl);
X is hydrogen or halogen;
Y is CQ (in which Q is hydrogen or halogen) or nitrogen or, when taken together with $R^1$, may form a group of the formula: $=C-OCH_2(R^9)H-$ (in which $R^9$ is hydrogen or lower alkyl and the carbon atom to which $R^9$ is attached links to the nitrogen atom in the pyridone ring);
Z is hydrogen or amino;
$\ell$ is an integer of 1 or 2;
m is an integer of 0 or 1; and
n is an integer of 1 or 2,
or a halt thereof.

2. A composition as claimed in Claim 1, wherein the composition is for the treatment or prophylaxis of mycoplasmal pneumonia in fowls; infectious coryza in fowls; mycoplasmal pneumonia in pigs; hemophilus pleuropneumonia in pigs; or mycoplasmal penumonia in cattle.

3. A veterinary composition as claimed in Claim 1 or Claim 2 which further comprises a veterinary acceptable diluent, carrier or excipient.

4. The use of a compound as defined in Claim 1 in the manufacture of a medicament for veterinary use.

5. A compound of the formula:

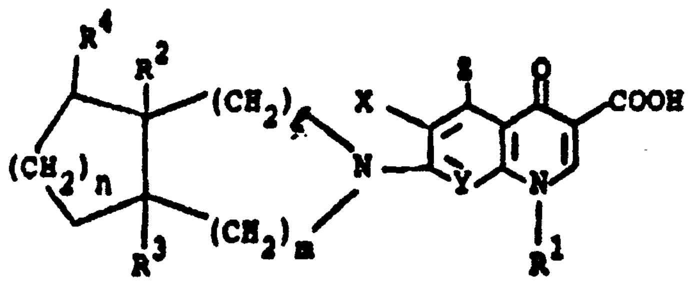

wherein

$R^1$ is lower alkyl, halo(lower)alkyl, cyclo(lower)alkyl or substituted or unsubstituted phenyl;

$R^2$ and $R^3$ are each hydrogen, hydroxyl, lower alkoxy, halogen or a group of the formula: $-CH_2N(R^5)R^6$ (in which $R^5$ and $R^6$ are each hydrogen, lower alkyl, lower alkenyl or hydroxy(lower) alkyl);

$R^4$ is hydrogen, oxo, hydroxyl, lower alkoxy, halogen or a group of the formula: $-CH_2N(R^7)R^8$ (in which $R^7$ and $R^8$ are each hydrogen or lower alkyl);

X is hydrogen or halogen;

Y is CQ (in which Q is hydrogen or halogen) or nitrogen or, when taken together with $R^1$, may form a group of the formula: $=C\text{-}OCH_2C(R^9)H\text{-}$ (in which $R^9$ is hydrogen or lower alkyl and the carbon atom to which $R^9$ is attached links to the nitrogen atom in the pyridone ring);

Z is hydrogen or amino;

$\ell$ is an integer of 1 or 2;

m is an integer of 0 or 1; and

n is an integer of 1 or 2,

or a salt thereof.

6. A process for the production of a compound as defined in Claim 1 comprising reacting a compound of the formula:

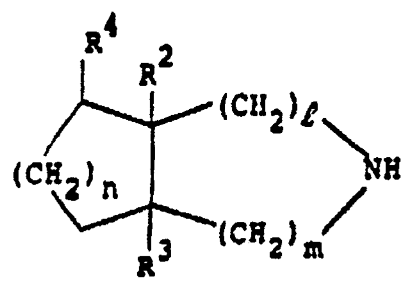

(III)

with a compound of the formula:

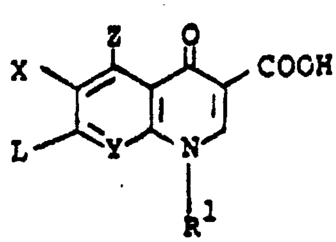

(II)

Claims for the following Contracting States: ES, GR

1. A process for the production of a veterinary composition for the treatment or prophylaxis of infectious diseases caused by infectious micoorganisms comprising admixing at least one compound of the formula:

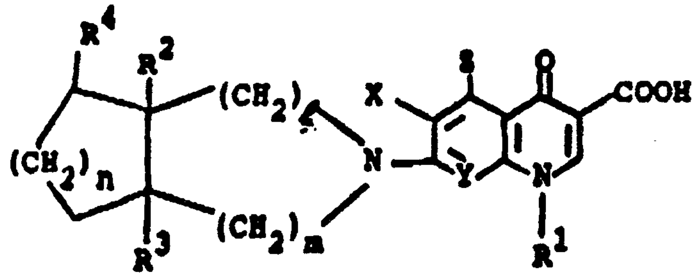

wherein

$R^1$ is lower alkyl, halo(lower)alkyl, cyclo(lower)alkyl or substituted or unsustituted phenyl;

$R^2$ and $R^3$ are each hydrogen, hydroxyl, lower alkoxy, halogen or a group of the formula: $-CH_2N(R^5)R^6$ (in which $R^5$ and $R^6$ are each hydrogen, lower alkyl, lower alkenyl or hydroxy(lower)alkyl);

$R^4$ is hydrogen, oxo, hydroxyl, lower alkoxy, halogen or a group of the formula: $-CH_2N(R^7)R^8$ (in which $R^7$ and $R^8$ are each hydrogen or lower alkyl);

X is hydrogen or halogen;

Y is CQ (in which Q is hydrogen or halogen) or nitrogen or, when taken together with $R^1$, may form a group of the formula: $=C-OCH_2C(R^9)H-$ (in which $R^9$ is hydrogen or lower alkyl and the carbon atom to which $R^9$ is attached links to the nitrogen atom in the pyridone ring); with one or more veterinarily acceptable excipients, diluents or carriers.

2. A process as claimed in Claim 1 wherein the composition is for the treatment or prophylaxis of mycoplasmal pneumonia in fowls; infectious coryza in fowls; mycoplasmal pneumonia in pigs; hemophilus pleuropneumonia in pigs; or mycoplasmal penumonia in cattle.

3. The use of a compound as defined in Claim 1 in the manufacture of a medicament for veterinary use.

4. A process for the production of a compound as defined in Claim 1 comprising reacting a compound of the formula:

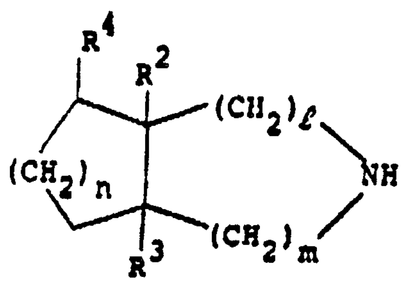

(III)

with a compound of the formula:

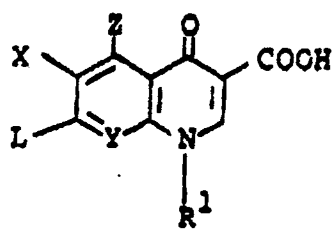

(II)